(19) Europäisches Patentamt / European Patent Office / Office européen des brevets

(11) **EP 1 817 419 B1**

(12) **EUROPEAN PATENT SPECIFICATION**

(45) Date of publication and mention of the grant of the patent:
**27.02.2013 Bulletin 2013/09**

(21) Application number: **05818939.0**

(22) Date of filing: **24.11.2005**

(51) Int Cl.:
***C12N 15/82*** *(2006.01)* ***C07K 14/415*** *(2006.01)*
***A01H 5/10*** *(2006.01)*

(86) International application number:
**PCT/EP2005/056202**

(87) International publication number:
**WO 2006/056590 (01.06.2006 Gazette 2006/22)**

(54) **PLANTS HAVING INCREASED YIELD AND A METHOD FOR MAKING THE SAME**

PFLANZEN MIT ERHÖHTEM ERTRAG UND VERFAHREN ZUR HERSTELLUNG DAVON

PLANTES À RENDEMENT AMÉLIORÉ ET PROCÉDÉ DE FABRICATION

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **25.11.2004 EP 04106065**
**01.12.2004 US 632273 P**

(43) Date of publication of application:
**15.08.2007 Bulletin 2007/33**

(73) Proprietor: **CropDesign N.V.**
**9052 Zwijnaarde-Gent (BE)**


(72) Inventor: **FRANKARD, Valerie**
**B-1410 Waterloo (BE)**

(74) Representative: **Mistry, Meeta et al**
**Greaves Brewster LLP**
**Copa House**
**Station Road**
**Cheddar, BS27 3AH (GB)**


(56) References cited:
**EP-A- 1 209 232 WO-A-02/33091**
**WO-A-99/00503 US-A1- 2010 175 146**


- **FORNARA FABIO ET AL: "Functional characterization of OsMADS18, a member of the AP1/SQUA subfamily of MADS box genes" PLANT PHYSIOLOGY (ROCKVILLE), vol. 135, no. 4, August 2004 (2004-08), pages 2207-2219, XP002325734 ISSN: 0032-0889 cited in the application**
- **MATSUOKA MAKOTO ET AL: "Expression of a rice homeobox gene causes altered morphology of transgenic plants" PLANT CELL, vol. 5, no. 9, 1993, pages 1039-1048, XP002325735 ISSN: 1040-4651 cited in the application**


Remarks:
The file contains technical information submitted after the application was filed and not included in this specification

EP 1 817 419 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


Printed by Jouve, 75001 PARIS (FR)

SEQUENCE LISTING

<110> CropDesign N.V.

<120> Plants having increased yield and a method for making the same

<130> CD-122-PCT

<150> EP 04106065.8
<151> 2004-11-25

<150> US 60/632,273
<151> 2004-12-01

<160> 22

<170> PatentIn version 3.3

<210> 1
<211> 750
<212> DNA
<213> Oryza sativa

<220>
<221> misc_feature
<223> OsMADS18 (AF091458)

<400> 1

```
atggggagag ggccggtgca gctgcggcgg atcgagaaca agataaacag gcaggtgacc      60
ttctccaagc ggaggaacgg gctgctgaag aaggcgcacg agatctccgt gctctgtgac     120
gccgacgtcg cgctcatcgt cttctccacc aagggcaagc tctacgagtt ctccagccac     180
tccagtatgg aagggatcct tgaacgctac cagcgttact cgtttgatga aagagccgta     240
ctggagccaa atactgagga ccaggaaaac tggggtgatg aatatggaat tttgaagtcc     300
aaactggatg cacttcagaa gagccaaagg caactcttag gtgaacaatt ggacacacta     360
acaataaaag aactccagca attggaacat caactggaat attctctgaa gcatataaga     420
tcaaaaaaga atcagcttct gtttgaatca atttctgagc ttcagaagaa ggaaaagtca     480
cttaaaaacc agaataatgt tctgcaaaag ctcatggaga cagaaaagga gaaaaacaat     540
gctataataa acactaaccg ggaggagcaa aatggagcaa caccaagcac atcatcacca     600
acaccagtga cggctccaga tcccatcccg acaacaaata acagtcaaag ccaaccaaga     660
ggatcagggg agtcagaagc tcaaccgtct ccggcacaag caggcaacag caagcttccg     720
ccatggatgc tccggacaag tcacacatga                                      750
```

<210> 2
<211> 249
<212> PRT
<213> Oryza sativa

<400> 2

```
Met Gly Arg Gly Pro Val Gln Leu Arg Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Asp Val Ala Leu Ile Val Phe
        35                  40                  45
```

```
Ser Thr Lys Gly Lys Leu Tyr Glu Phe Ser Ser His Ser Ser Met Glu
    50                  55                  60
Gly Ile Leu Glu Arg Tyr Gln Arg Tyr Ser Phe Asp Glu Arg Ala Val
65                  70                  75                  80
Leu Glu Pro Asn Thr Glu Asp Gln Glu Asn Trp Gly Asp Glu Tyr Gly
                85                  90                  95
Ile Leu Lys Ser Lys Leu Asp Ala Leu Gln Lys Ser Gln Arg Gln Leu
            100                 105                 110
Leu Gly Glu Gln Leu Asp Thr Leu Thr Ile Lys Glu Leu Gln Gln Leu
        115                 120                 125
Glu His Gln Leu Glu Tyr Ser Leu Lys His Ile Arg Ser Lys Lys Asn
    130                 135                 140
Gln Leu Leu Phe Glu Ser Ile Ser Glu Leu Gln Lys Lys Glu Lys Ser
145                 150                 155                 160
Leu Lys Asn Gln Asn Asn Val Leu Gln Lys Leu Met Glu Thr Glu Lys
                165                 170                 175
Glu Lys Asn Asn Ala Ile Ile Asn Thr Asn Arg Glu Glu Gln Asn Gly
            180                 185                 190
Ala Thr Pro Ser Thr Ser Ser Pro Thr Pro Val Thr Ala Pro Asp Pro
        195                 200                 205
Ile Pro Thr Thr Asn Asn Ser Gln Ser Gln Pro Arg Gly Ser Gly Glu
    210                 215                 220
Ser Glu Ala Gln Pro Ser Pro Ala Gln Ala Gly Asn Ser Lys Leu Pro
225                 230                 235                 240
Pro Trp Met Leu Arg Thr Ser His Thr
                245
```

<210> 3
<211> 756
<212> DNA
<213> Zea mays

<220>
<221> misc_feature
<223> ZMM28 (or m28) (AJ430695)

<400> 3

```
atggggcggg ggccggtgca gctgcgccgg atcgagaaca agatcaaccg ccaggtgacc     60
ttctccaagc gccggaacgg gctgctgaag aaggcccacg agatctccgt gctctgcgac    120
gcagaggtcg cgctcatcgt cttctccact aaggggaagc tctacgagta ctctagccat    180
tccagcatgg aaggcattct tgagcgttac cagcgttact catttgaaga aagggcagta    240
cttaacccaa gtattgaaga ccaggcaaat tggggagatg aatatgtccg gttaaaatcc    300
aaacttgatg cacttcagaa gagtcaaagg cagctgttag gagaacaatt gagttcactg    360
accataaaag aactccagca actggagcaa caactggaca gttctttgaa gcatattagg    420
tcaagaaaga atcagctcat gttcgattca atttccgcgc ttcagaaaaa ggagaaagca    480
cttacagatc aaaacggtgt cctgcaaaag ttcatggagg cagagaagga gaaaaacaag    540
gctttgatga acgcgcagct ccgggagcag caaaatggag catcaacaag ctccccatca    600
ctttcaccac caatagttcc agattccatg ccaactctaa atatagggcc atgtcaacat    660
agaggggcag cagaatctga gtctgaaccg tctcctgctc ctgcacaagc aaacaggggc    720
aacctgccac catggatgct ccgcactgtc aagtaa                              756
```

<210> 4
<211> 251
<212> PRT
<213> Zea mays

<400> 4

```
Met Gly Arg Gly Pro Val Gln Leu Arg Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Leu Ile Val Phe
        35                  40                  45
Ser Thr Lys Gly Lys Leu Tyr Glu Tyr Ser Ser His Ser Ser Met Glu
    50                  55                  60
Gly Ile Leu Glu Arg Tyr Gln Arg Tyr Ser Phe Glu Glu Arg Ala Val
65                  70                  75                  80
Leu Asn Pro Ser Ile Glu Asp Gln Ala Asn Trp Gly Asp Glu Tyr Val
                85                  90                  95
Arg Leu Lys Ser Lys Leu Asp Ala Leu Gln Lys Ser Gln Arg Gln Leu
            100                 105                 110
Leu Gly Glu Gln Leu Ser Ser Leu Thr Ile Lys Glu Leu Gln Gln Leu
        115                 120                 125
Glu Gln Gln Leu Asp Ser Ser Leu Lys His Ile Arg Ser Arg Lys Asn
    130                 135                 140
Gln Leu Met Phe Asp Ser Ile Ser Ala Leu Gln Lys Lys Glu Lys Ala
145                 150                 155                 160
Leu Thr Asp Gln Asn Gly Val Leu Gln Lys Phe Met Glu Ala Glu Lys
                165                 170                 175
Glu Lys Asn Lys Ala Leu Met Asn Ala Gln Leu Arg Glu Gln Gln Asn
            180                 185                 190
Gly Ala Ser Thr Ser Ser Pro Ser Leu Ser Pro Pro Ile Val Pro Asp
        195                 200                 205
Ser Met Pro Thr Leu Asn Ile Gly Pro Cys Gln His Arg Gly Ala Ala
    210                 215                 220
Glu Ser Glu Ser Glu Pro Ser Pro Ala Pro Ala Gln Ala Asn Arg Gly
225                 230                 235                 240
Asn Leu Pro Pro Trp Met Leu Arg Thr Val Lys
                245                 250
```

<210> 5
<211> 840
<212> DNA
<213> Lolium perenne

<220>
<221> misc_feature
<223> MADS3 (AY198328)

<400> 5

```
atggggcgcg ggccggtgca gctgcggcgg atcgagaaca agataaaccg ccaggtcacc     60
ttctccaagc gccggagcgg gctgctcaag aaggcgcacg agatctccgt gctctgcgac    120
gctgaggtcg cgctcatcgt attctccacc aagggaaagc tctacgagta ctccagccag    180
gacagtaaca tggatgtcat tcttgaacgt taccagcgtt actcattcga agaaagagct    240
atagtggatc aaaatattgg aggccaggca aattggggag atgaatttgg aagtttgaaa    300
attaaactcg atgcactcca gaagagtcaa aggcaactct taggtgaaca attggaccca    360
ctgaccacaa aagaacttca acaattggaa cagcagctag atagttcttt gaagcacata    420
aggtcaagaa agaatcagct tctgtttgag tcaatatctg agcttcagaa gaaggagaag    480
```

```
tcacttaaag atcagaatgg cgtcctgcag aagcacctcg tggagacaga aaaggagaaa    540
agtaacgttt tatcgaatat tcatcaccga gagcagacga atggagcagc aaatattcat    600
cgccgggagc agatgaatga aacaacacat attcataacc aggagcagct caatggagca    660
acaacaagct caccgtcacc tacaccagtg gcggtcctag attccgtggc aactctaaat    720
attgggtcat ctcaatctag agaagcagca ggagaggagc cagaatctca gccgtccccg    780
gcacaagcaa acagcggcaa gctaccacca tggatgctcc gcaccatcag taacagatga    840
```

<210> 6
<211> 279
<212> PRT
<213> Lolium perenne

<400> 6

```
Met Gly Arg Gly Pro Val Gln Leu Arg Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Ala Leu Ile Val Phe
        35                  40                  45
Ser Thr Lys Gly Lys Leu Tyr Glu Tyr Ser Ser Gln Asp Ser Asn Met
    50                  55                  60
Asp Val Ile Leu Glu Arg Tyr Gln Arg Tyr Ser Phe Glu Glu Arg Ala
65                  70                  75                  80
Ile Val Asp Gln Asn Ile Gly Gly Gln Ala Asn Trp Gly Asp Glu Phe
                85                  90                  95
Gly Ser Leu Lys Ile Lys Leu Asp Ala Leu Gln Lys Ser Gln Arg Gln
            100                 105                 110
Leu Leu Gly Glu Gln Leu Asp Pro Leu Thr Thr Lys Glu Leu Gln Gln
        115                 120                 125
Leu Glu Gln Gln Leu Asp Ser Ser Leu Lys His Ile Arg Ser Arg Lys
    130                 135                 140
Asn Gln Leu Leu Phe Glu Ser Ile Ser Glu Leu Gln Lys Lys Glu Lys
145                 150                 155                 160
Ser Leu Lys Asp Gln Asn Gly Val Leu Gln Lys His Leu Val Glu Thr
                165                 170                 175
Glu Lys Glu Lys Ser Asn Val Leu Ser Asn Ile His His Arg Glu Gln
            180                 185                 190
Thr Asn Gly Ala Ala Asn Ile His Arg Arg Glu Gln Met Asn Glu Thr
        195                 200                 205
Thr His Ile His Asn Gln Glu Gln Leu Asn Gly Ala Thr Thr Ser Ser
    210                 215                 220
Pro Ser Pro Thr Pro Val Ala Val Leu Asp Ser Val Ala Thr Leu Asn
225                 230                 235                 240
Ile Gly Ser Ser Gln Ser Arg Glu Ala Ala Gly Glu Glu Pro Glu Ser
                245                 250                 255
Gln Pro Ser Pro Ala Gln Ala Asn Ser Gly Lys Leu Pro Pro Trp Met
            260                 265                 270
Leu Arg Thr Ile Ser Asn Arg
        275
```

<210> 7
<211> 798
<212> DNA
<213> Hordeum vulgare

<220>
<221> misc_feature

<223> m3 (AJ249143)

<400> 7

```
atggggcgcg ggccggtgca gctgcggcgg atcgagaaca agataaaccg gcaggtgacc    60
ttctccaagc ggcgcagcgg gctgcttaag aaggcgcacg agatctccgt gctctgcgac   120
gccgaggtcg cgctcatcgt cttctccacc aagggcaagc tatacgagta ctccagccag   180
gacagtagca tggatgtgat tcttgaacgt taccaacgtt actcatttga agaaagagct   240
gtactggatc caagtactgg agaccaggca aattggggag acgaatatgg aagtttgaag   300
ataaaactcg atgcactcca gaagagtcaa aggcaactct taggtgaaca attggaccca   360
ctcaccacaa aagaacttca acagttggaa caacagcttg atagttcttt gaagcacata   420
aggtcaagaa agaatcaact tctctttgag tcaatatctg agcttcaaaa gaaggagaaa   480
tcattaaaag atcagaatgg cgtcctgcag aagcacctcg tagagacaga aaaggagaaa   540
aataacgttt tgtcaaatat tcatcaccgg gagcagttga atgaagcaac aaatattcat   600
caccaggagc agctgagtgg agcaacaaca agctcaccgt cacctacacc accgacggcc   660
caagattcca tggcacctcc aaatattggg ccatatcaat ctagaggagg aggggatcca   720
gaacctcagc cgtcaccagc acaagcaaac aacagcaatc tgccaccgtg gatgctccgc   780
accatcggca acagatga                                                798
```

<210> 8
<211> 264
<212> PRT
<213> Hordeum vulgare

<400> 8

```
Met Gly Arg Gly Pro Val Gln Leu Arg Arg Ile Glu Asn Lys Ile Asn
1               5                   10                  15
Arg Gln Val Thr Phe Ser Lys Arg Arg Ser Gly Leu Leu Lys Lys Ala
            20                  25                  30
His Glu Ile Ser Val Leu Cys Asp Ala Glu Val Ala Leu Ile Val Phe
        35                  40                  45
Ser Thr Lys Gly Lys Leu Tyr Glu Tyr Ser Ser Gln Asp Ser Ser Met
    50                  55                  60
Asp Val Ile Leu Glu Arg Tyr Gln Arg Tyr Ser Phe Glu Glu Arg Ala
65                  70                  75                  80
Val Leu Asp Pro Ser Thr Gly Asp Gln Ala Asn Trp Gly Asp Glu Tyr
                85                  90                  95
Gly Ser Leu Lys Ile Lys Leu Asp Ala Leu Gln Lys Ser Gln Arg Gln
            100                 105                 110
Leu Leu Gly Glu Gln Leu Asp Pro Leu Thr Thr Lys Glu Leu Gln Gln
        115                 120                 125
Leu Glu Gln Gln Leu Asp Ser Ser Leu Lys His Ile Arg Ser Arg Lys
    130                 135                 140
Asn Gln Leu Leu Phe Glu Ser Ile Ser Glu Leu Gln Lys Lys Glu Lys
145                 150                 155                 160
Ser Leu Lys Asp Gln Asn Gly Val Leu Gln Lys His Leu Val Glu Thr
                165                 170                 175
Glu Lys Glu Lys Asn Asn Val Leu Ser Asn Ile His His Arg Glu Gln
            180                 185                 190
Leu Asn Glu Ala Thr Asn Ile His His Gln Glu Gln Leu Ser Gly Ala
        195                 200                 205
Thr Thr Ser Ser Pro Ser Pro Thr Pro Pro Thr Ala Gln Asp Ser Met
```

```
            210                 215                 220
        Ala Pro Pro Asn Ile Gly Pro Tyr Gln Ser Arg Gly Gly Gly Asp Pro
        225                 230                 235                 240
        Glu Pro Gln Pro Ser Pro Ala Gln Ala Asn Asn Ser Asn Leu Pro Pro
                        245                 250                 255
        Trp Met Leu Arg Thr Ile Gly Asn
                    260
```

<210> 9
<211> 727
<212> DNA
<213> Saccharum officinarum

<220>
<221> misc_feature
<223> ZMM28-like partial DNA (EST CA285442)

<220>
<221> misc_feature
<222> (620)..(620)
<223> n is a, c, g, or t

<220>
<221> misc_feature
<222> (662)..(662)
<223> n is a, c, g, or t

<220>
<221> misc_feature
<222> (689)..(689)
<223> n is a, c, g, or t

<400> 9

```
cgcaccactc gagtcgagag acgagagcca cctgatccat ccttccccgt cttcctcttc      60
ccacacgtcc ccatcgccat tgctgcgccg agagtgagcg ggagagggta ggtgtcgagg     120
cggcggagat ggggagcggg ccggtgcagc tgcgccggat cgagaacaag atcaaccgcc     180
aggtgacctt ctccaagcgc cggaacgggc tgctgaagaa ggcccacgag atctccgtgc     240
tctgcgatgc agaggtcgcg ctcatcgtct tctccactaa ggggaagctc tacgagtact     300
ctagccattc cagcatggaa ggcattcttg agcgttacca gcggtactca tttgaagaaa     360
gagcagtact tgacccaagt attgaagacc aggcagattg gggagatgaa tatgtccggt     420
taaaatccaa acttgatgca cttcagaaga gtcaaaggca tctgctagga gaacaattgg     480
attcactgac cataaaagaa ctccagcaac tggagcaaca attggacagt tctttgaagc     540
atattaggtc aagaaagaat cagctcatgt tcgattcaat ttctgagctt cagaaaaagg     600
agaaagcact tacagatcan aatggtgtcc tgcaaaagct catggaggca gagaaagaga     660
anaacaatgc tttaatgaac gcacacctnc gggagcagca aaatggagca tcaacaggct     720
ccccata                                                               727
```

<210> 10
<211> 680
<212> DNA
<213> Saccharum officinarum

<220>
<221> misc_feature
<223> ZMM28-like partial DNA (EST CA200888)

<400> 10

ttcttgagcg ttaccagcgg tactcatttg aagaaagagc agtacttgac ccaagtattg 60
aagaccaggc agattgggga gatgaatatg tccagttaaa atccaaactt gatgcacttc 120
agaagagtca aagacatctg ttaggagaac aattggattc actgaccata aaagaactcc 180
agcaactgga gcaacaattg gacagttctt tgaagcatat taggtcaaga aagaatcagc 240
tcatgttcga ttcaatttcc gagcttcaga aaaaggagaa agcacttaca gatcaaaatg 300
gtgtcctgca aaagctcatg gaggcagaga aggagaaaaa caatgcttta atgaacgcac 360
acctccggga gcagcaaaat gtagcatcaa caagctcccc atcactgtca ccaccaatgg 420
ttccagattc catgccaact ctaaatattg ggtcatgtca acctagaggg ccaggagaat 480
ctgagcctga accgtctcct gctcctgtac aagcaaacag cggcaacctg ccaccatgga 540
tgctccgcac tgtcagtaac agatgagctc ttcccaatgc agttttgcgg ctgaacacga 600
cagcaacaca cctgccaccg acacgccacg gtgacccagg catcaaccga tgcatgatcc 660
ctctcgatcc tgtgatccaa 680

<210> 11
<211> 483
<212> DNA
<213> Saccharum officinarum

<220>
<221> misc_feature
<223> ZMM28-like partial DNA (EST CA247266)

<400> 11

cttattttgc agcatggaag gcattcttga gcgttaccag cggtactcat ttgaagaaag 60
agcagtactt gacccaagta ttgaagacca ggcagattgg ggagatgaat atgtccagtt 120
aaaatccaaa cttgatgcac ttcagaagag tcaaagacat ctgttaggag aacaattgga 180
ttcactgacc ataaaagaac tccagcaact ggagcaacaa ttggacagtt ctttgaagca 240
tattaggtca agaaagaatc agctcatgtt cgattcaatt tccgagcttc agaaaaagga 300
gaaagcactt acagatcaaa atggtgtcct gcaaaagctc atggaggcag agaaggagaa 360
aaacaatgct ttaatgaacg cacacctccg ggagcagcaa aatgtagcat caacaagctc 420
cccatcactg tcaccaccaa tggttccaga ttccatgcca actctaaata ttgggatgtc 480
aac 483

<210> 12
<211> 714
<212> DNA
<213> Saccharum officinarum

<220>
<221> misc_feature
<223> ZMM28-like partial DNA (EST CA282968)

<400> 12

cagtacttga cccaagtatt aaagaccagg cagattgggg agatgaatat gtccggttaa 60
aatccaaact tgatgcactt cagaagagtc aaaggcatct gttaggagaa caattggatt 120
cactgaccat aaaagaactc cagcaactgg agcaacaatt ggacagttct ttgaagcata 180
ttaggtcaag aaagaatcag ctcatgttcg attcaatttc cgagcttcag aaaaaggaga 240
aagcacttac agatcaaaat ggtgtcctgc aaaagctcat ggaggcagag aaggagaaaa 300
acaatgcttt aatgaacgca cacctccggg agcagcaaaa tgtagcatca acaagctccc 360
catcactgtc accaccaatg gttccagatt ccatgccaac tctaaatatt gggtcatgtc 420
aacctagagg gccaggagaa tctgagcctg aaccgtctcc tgctcctgta caagcaaaca 480

```
gcggcaacct gccaccatgg atgctccgca ctgtcagtaa cagatgagct cttcccaatg    540
cagttttgcg gctgaacacg acagcaacac acctgccacc gacacgccac ggtgaaccag    600
gcatcaaccg atgcatgatc cctctcgatc ctgtgatcca ataccgggcc gtagtactac    660
taattaaagt ccaggccccg ctcaaattgc tttacatggt tgattgaaag ttct          714
```

<210> 13
<211> 650
<212> DNA
<213> Saccharum officinarum

<220>
<221> misc_feature
<223> ZMM28-like partial DNA (EST CA299090)

<220>
<221> misc_feature
<222> (626)..(626)
<223> n is a, c, g, or t

<400> 13

```
gaagctctac gagtactcta gccattccag catggaaggc attcttgagc gttaccagcg     60
gtgctcattt gaagaaagag cagtacttga cccaagtatt gaagaccagg cagattgggg    120
agatgaatat gtccggttaa aatccaaact tgatgcactt cagaagagtc aaaggcatct    180
gttaggagaa caattggatt cactgaccat aaaagaactc cagcaactgg agcaacaatt    240
ggacagttct ttgaagcata ttaggtcaag aaagaatcag ctcatgttcg attcaatttc    300
cgagcttcag aaaaaggaga aagcacttac agatcaaaat ggtgtcctgc aaaagctcat    360
ggaggcagag aaggagaaaa acaatgcttt aatgaacgca cacctccggg agcagcaaaa    420
tggagcatca acaagctccc catcactgtc accaccaatg gttccagatt ccatgccaac    480
tctaaatatt gggtcatgtc aacctagagg gccaggagaa tctgagcctg aaccgtctcc    540
tgctcctgta caagcaaaca gcgggaacct gccaccatgg atactccgca ctgtcagtaa    600
cagatgagct ctttccaatg cagttntgcg gctgaactcg acagcaacac                650
```

<210> 14
<211> 762
<212> DNA
<213> Saccharum officinarum

<220>
<221> misc_feature
<223> ZMM28-like reconstituted DNA version 1

<400> 14

```
atggggagcg ggccggtgca gctgcgccgg atcgagaaca agatcaaccg ccaggtgacc     60
ttctccaagc gccggaacgg gctgctgaag aaggcccacg agatctccgt gctctgcgat    120
gcagaggtcg cgctcatcgt cttctccact aaggggaagc tctacgagta ctctagccat    180
tccagcatgg aaggcattct tgagcgttac cagcggtact catttgaaga aagagcagta    240
cttgacccaa gtattgaaga ccaggcagat tggggagatg aatatgtccg gttaaaatcc    300
aaacttgatg cacttcagaa gagtcaaagg catctgttag gagaacaatt ggattcactg    360
accataaaag aactccagca actggagcaa caattggaca gttctttgaa gcatattagg    420
tcaagaaaga atcagctcat gttcgattca atttccgagc ttcagaaaaa ggagaaagca    480
cttacagatc aaaatggtgt cctgcaaaag ctcatggagg cagagaagga gaaaaacaat    540
gctttaatga acgcacacct ccgggagcag caaaatggag catcaacaag ctccccatca    600
ctgtcaccac caatggttcc agattccatg ccaactctaa atattgggtc atgtcaacct    660
agagggccag gagaatctga gcctgaaccg tctcctgctc ctgtacaagc aaacagcggc    720
```

aacctgccac catggatgct ccgcactgtc agtaacagat ga 762

<210> 15
<211> 253
<212> PRT
<213> Saccharum officinarum

<220>
<221> MISC_FEATURE
<223> ZMM28-like reconstituted protein version 1

<400> 15

```
Met Gly Ser Gly Pro Val Gln Leu Arg Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Leu Ile Val Phe
        35                  40                  45
Ser Thr Lys Gly Lys Leu Tyr Glu Tyr Ser Ser His Ser Ser Met Glu
    50                  55                  60
Gly Ile Leu Glu Arg Tyr Gln Arg Tyr Ser Phe Glu Glu Arg Ala Val
65                  70                  75                  80
Leu Asp Pro Ser Ile Glu Asp Gln Ala Asp Trp Gly Asp Glu Tyr Val
                85                  90                  95
Arg Leu Lys Ser Lys Leu Asp Ala Leu Gln Lys Ser Gln Arg His Leu
            100                 105                 110
Leu Gly Glu Gln Leu Asp Ser Leu Thr Ile Lys Glu Leu Gln Gln Leu
        115                 120                 125
Glu Gln Gln Leu Asp Ser Ser Leu Lys His Ile Arg Ser Arg Lys Asn
    130                 135                 140
Gln Leu Met Phe Asp Ser Ile Ser Glu Leu Gln Lys Lys Glu Lys Ala
145                 150                 155                 160
Leu Thr Asp Gln Asn Gly Val Leu Gln Lys Leu Met Glu Ala Glu Lys
                165                 170                 175
Glu Lys Asn Asn Ala Leu Met Asn Ala His Leu Arg Glu Gln Gln Asn
            180                 185                 190
Gly Ala Ser Thr Ser Ser Pro Ser Leu Ser Pro Pro Met Val Pro Asp
        195                 200                 205
Ser Met Pro Thr Leu Asn Ile Gly Ser Cys Gln Pro Arg Gly Pro Gly
    210                 215                 220
Glu Ser Glu Pro Glu Pro Ser Pro Ala Pro Val Gln Ala Asn Ser Gly
225                 230                 235                 240
Asn Leu Pro Pro Trp Met Leu Arg Thr Val Ser Asn Arg
                245                 250
```

<210> 16
<211> 762
<212> DNA
<213> Saccharum officinarum

<220>
<221> misc_feature
<223> ZMM28-like reconstituted DNA version 2

<400> 16

```
atggggagcg ggccggtgca gctgcgccgg atcgagaaca agatcaaccg ccaggtgacc    60
ttctccaagc gccggaacgg gctgctgaag aaggcccacg agatctccgt gctctgcgat   120
gcagaggtcg cgctcatcgt cttctccact aaggggaagc tctacgagta ctctagccat   180
tccagcatgg aaggcattct tgagcgttac cagcggtact catttgaaga aagagcagta   240
cttgacccaa gtattgaaga ccaggcagat tggggagatg aatatgtccg gttaaaatcc   300
aaacttgatg cacttcagaa gagtcaaagg catctgttag gagaacaatt ggattcactg   360
accataaaag aactccagca actggagcaa caattggaca gttctttgaa gcatattagg   420
tcaagaaaga atcagctcat gttcgattca atttccgagc ttcagaaaaa ggagaaagca   480
cttacagatc aaaatggtgt cctgcaaaag ctcatggagg cagagaagga gaaaaacaat   540
gctttaatga acgcacacct ccgggagcag caaaatgtag catcaacaag ctccccatca   600
ctgtcaccac caatggttcc agattccatg ccaactctaa atattgggtc atgtcaacct   660
agagggccag gagaatctga gcctgaaccg tctcctgctc ctgtacaagc aaacagcggc   720
aacctgccac catggatgct ccgcactgtc agtaacagat ga                      762
```

<210> 17
<211> 253
<212> PRT
<213> Saccharum officinarum

<220>
<221> MISC_FEATURE
<223> ZMM28-like reconstituted protein version 2

<400> 17

```
Met Gly Ser Gly Pro Val Gln Leu Arg Arg Ile Glu Asn Lys Ile Asn
1               5                   10                  15
Arg Gln Val Thr Phe Ser Lys Arg Arg Asn Gly Leu Leu Lys Lys Ala
            20                  25                  30
His Glu Ile Ser Val Leu Cys Asp Ala Glu Val Ala Leu Ile Val Phe
        35                  40                  45
Ser Thr Lys Gly Lys Leu Tyr Glu Tyr Ser Ser His Ser Ser Met Glu
    50                  55                  60
Gly Ile Leu Glu Arg Tyr Gln Arg Tyr Ser Phe Glu Glu Arg Ala Val
65                  70                  75                  80
Leu Asp Pro Ser Ile Glu Asp Gln Ala Asp Trp Gly Asp Glu Tyr Val
                85                  90                  95
Arg Leu Lys Ser Lys Leu Asp Ala Leu Gln Lys Ser Gln Arg His Leu
            100                 105                 110
Leu Gly Glu Gln Leu Asp Ser Leu Thr Ile Lys Glu Leu Gln Gln Leu
        115                 120                 125
Glu Gln Gln Leu Asp Ser Ser Leu Lys His Ile Arg Ser Arg Lys Asn
    130                 135                 140
Gln Leu Met Phe Asp Ser Ile Ser Glu Leu Gln Lys Lys Glu Lys Ala
145                 150                 155                 160
Leu Thr Asp Gln Asn Gly Val Leu Gln Lys Leu Met Glu Ala Glu Lys
                165                 170                 175
Glu Lys Asn Asn Ala Leu Met Asn Ala His Leu Arg Glu Gln Gln Asn
            180                 185                 190
Val Ala Ser Thr Ser Ser Pro Ser Leu Ser Pro Pro Met Val Pro Asp
        195                 200                 205
Ser Met Pro Thr Leu Asn Ile Gly Ser Cys Gln Pro Arg Gly Pro Gly
    210                 215                 220
Glu Ser Glu Pro Glu Pro Ser Pro Ala Pro Val Gln Ala Asn Ser Gly
225                 230                 235                 240
Asn Leu Pro Pro Trp Met Leu Arg Thr Val Ser Asn Arg
                245                 250
```

<210> 18
<211> 8
<212> PRT
<213> Artificial sequence

<220>
<223> amino acid consensus sequence monocot RT C-terminal motif

<400> 18

Leu Pro Pro Trp Met Leu Arg Thr
1 5

<210> 19
<211> 8
<212> PRT
<213> Artificial sequence

<220>
<223> amino acid consensus sequence monocot SH C-terminal motif

<400> 19

Leu Pro Pro Trp Met Leu Ser His
1 5

<210> 20
<211> 50
<212> DNA
<213> Artificial sequence

<220>
<223> primer prm05821

<400> 20
ggggacaagt ttgtacaaaa aagcaggctt aaacaatggg gagagggccg 50

<210> 21
<211> 50
<212> DNA
<213> Artificial sequence

<220>
<223> primer prm05822

<400> 21
ggggaccact ttgtacaaga aagctgggtt gagtggagtg acgtttgaga 50

<210> 22
<211> 1042
<212> DNA
<213> Oryza sativa

<220>
<221> misc_feature
<223> OSH1 promoter

<400> 22

```
ggggagttag gaaccttgac atacaaccaa tgataccatt ttttttcaag cagctagagg      60
tacagaggtt catatttttt agatggctca ttagtgatat tttagtcaaa aatttcagaa     120
gtacggccac gggtgatggc ctgaactaat attttattcg aggtgccgct acatcatcgt     180
ctaaagtaca cgcaagattc aacggaaaaa agaaaccgat cgatcgagat cagttagtta     240
atgaaataac tagatcaact catgtcgtca aaaacaaaag atgctcatct atggacaaca     300
cacgctgatg attcgatcat caaacaaagg tggtagtagt agtaaagcgt atcgtgtttc     360
tcatcagaaa gaagaattaa agaaaaaact aatcccgtct cgcgagccag agaaaattcc     420
ctacaaaagc cactcctttg atttgacatg caaaagcaag gctccactcc tctactaccc     480
tacaactaca caacactgtc tctctatctc caaaggcagt agctgtattg gcttccagct     540
tttcctctct acctctaatg atagcttgga gcaagttcaa tagtatagct aactactagc     600
tctaattcat ctataatcaa tctaatagct tattcataca atagttatat actacattat     660
taatatctgg tcccatctat catacacact gagtctgtgc tatagctgac tacaaatctg     720
tagcccgctg ctcttctctc ttcatttatc ttcttaaaat atatttgcag ctggcttatg     780
gcttatagct tgatattgag agggagagga gtgagagcta gctcagctca gctcaaggta     840
aacaacaagg cacactcttc ctacctcttc tccggttctt ccttttttcct ctttctcttc     900
gtccaagaac ttcacctcaa tagctcgagc tacggcctaa cttttgcgtt gcgcaggaga     960
gctcgatcgc tgcaccaata cttcactgga gatcgcctag ctgcagctag ctagcttatc    1020
ctgcgtgtct tgagcttctc tc                                            1042
```

## Description

The present invention relates generally to the field of molecular biology and concerns a method for increasing plant yield relative to corresponding wild type plants. More specifically, the present invention concerns a method for increasing plant seed yield by increasing activity in a plant of an *Oryza sativa* (Os)MADS18-like polypeptide or a homologue thereof. The present invention also concerns plants having increased activity of an OsMADS18-like polypeptide or a homologue thereof, which plants have increased seed yield relative to corresponding wild type plants. The invention also provides constructs useful in the methods of the invention.

The ever-increasing world population and the dwindling supply of arable land available for agriculture fuel research towards improving the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is yield. Yield is normally defined as the measurable produce of economic value from a crop. This may be defined in terms of quantity and/or quality. Yield is directly dependent on several factors, for example, the number and size of the organs, plant architecture (for example, the number of branches), seed production and more. Root development, nutrient uptake and stress tolerance may also be important factors in determining yield. Optimizing one of the abovementioned factors may therefore increase crop yield.

The ability to increase plant yield would have many applications in areas such as agriculture, including in the production of ornamental plants, arboriculture, horticulture and forestry.

It has now been found that increasing activity in a plant of an OsMADS18-like polypeptide gives plants having increased seed yield relative to corresponding wild type plants.

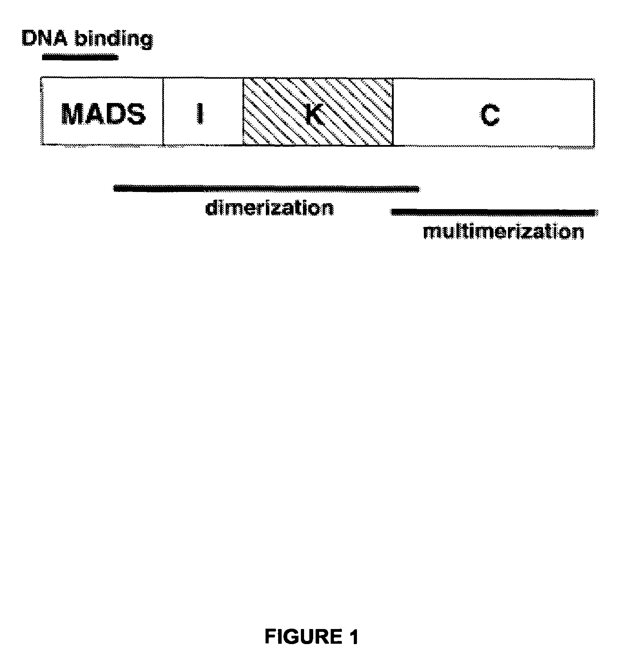

The MADS-box genes (MCM1 in yeast, Agamous and Deficiens in plants, and SRF (serum response factor) in humans) constitute a large gene family of eukaryotic transcriptional regulators involved in diverse aspects of yeast, plant and animal development. MADS-box genes encode a strongly conserved MADS domain responsible for DNA binding to specific boxes in the regulatory region of their target genes. The gene family can be divided into two main lineages, type I and type II. Type II genes are also named MIKC-type proteins, referring to the four functional domains they possess (see Figure 1, from Jack (2001) Plant Molec Biol 46: 515-520):

- MADS for DNA binding, about 60 amino acids (highly conserved) located at the N-terminal end of the protein;
- I for intervening domain (less conserved), involved in the selective formation of MADS dimer;
- K for keratin domain (well conserved) responsible for dimerization;
- C for C-terminal region (variable in sequence and length) involved in transcriptional activation, or in the formation of a multimeric transcription factor complex.

Over 100 MADS-box genes have been identified in *Arabidopsis,* and have been phylogenetically classified into 12 clades, each clade having specific deviations from the MADS consensus (Thiessen et al. (1996) J Mol Evol 43: 484-516). OsMADS18 (formerly called FDRMADS7 or OsMADS28) belongs to the SQUA clade (for SQUAMOSA, from *Antirrhinum majus),* along with the following *Arabidopsis* genes: FUL/Agl8 (FRUITFULL), CAL/Agl10 (CAULIFLOWER), AP1/Agl7 (APETALA1) and the less characterized MADS AGL79. Genes of the SQUA clade are A function organ identity genes with reference to the ABC floral organ identity specification model as proposed by Coen and Meyerowitz in 1991 (Nature 353: 31-7). Rice possesses four A-group genes: OsMADS14, OsMADS15, OsMADS18, and OsMADS20, each being member of the SQUA clade.

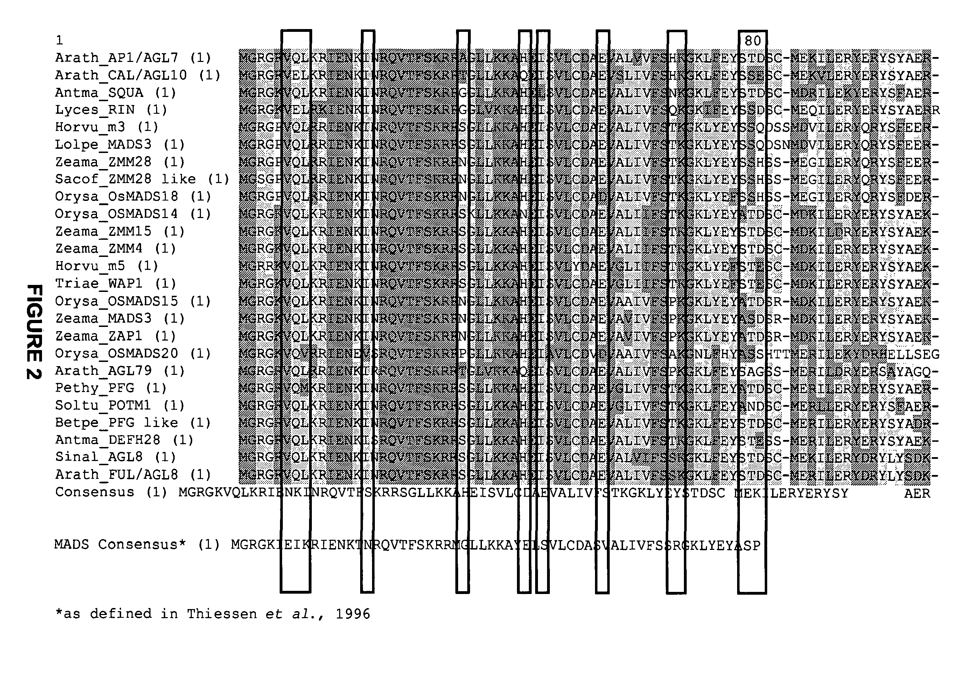

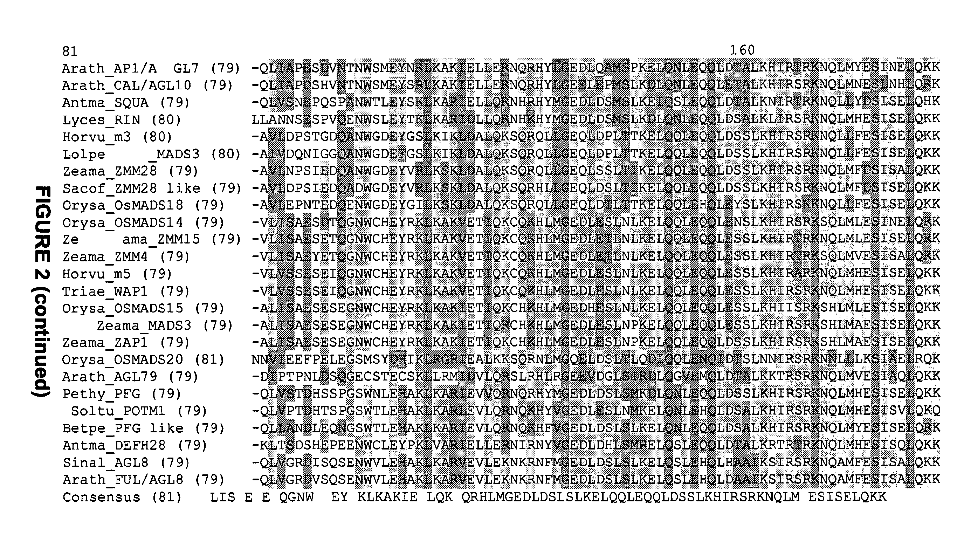

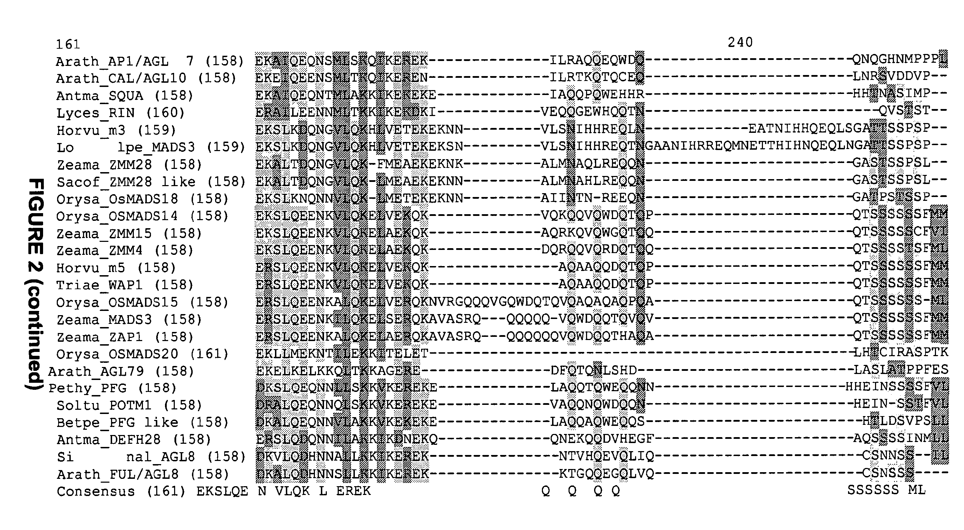

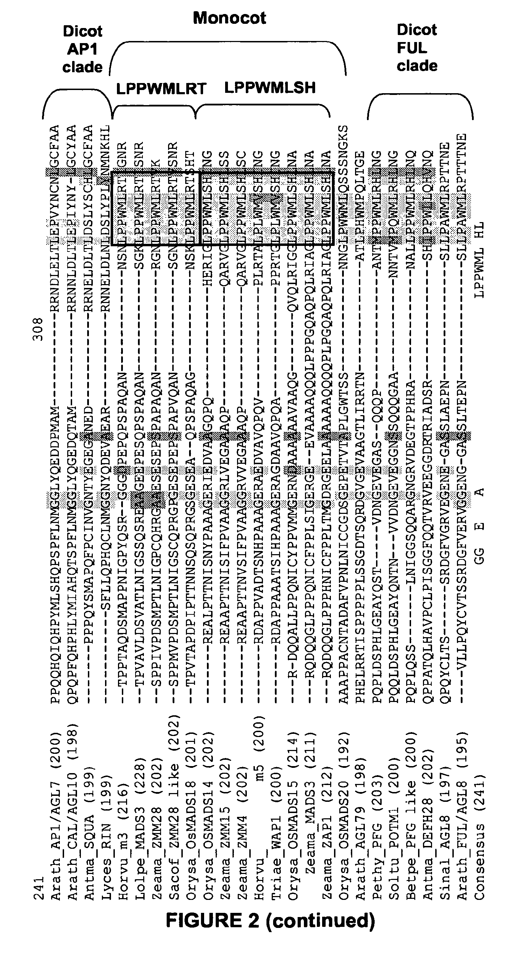

The SQUA clade in dicots is subdivided into two subgroups, the AP1 and the FUL subdades. These subclades diverge essentially with respect to the specific amino acid motifs located at the C-terminus of their respective proteins (Litt and Irish (2003) Genetics 165:821-833). In addition to the presence of a specific AP1 amino acid motif, the dicot AP1 clade related proteins usually comprise a farnesylation motif at their C-terminus (this motif is CAAX, where C is cysteine, A is usually an aliphatic amino acid, and X is methionine, glutamine, serine, cysteine or alanine). In monocots, the SQUA clade proteins are also subdivided into two main groups, which may be distinguished based on conserved C-terminal motifs located within the last 15 amino acids of the proteins: LPPWMLRT (SEQ ID NO: 18) and LPPWMLSH (SEQ ID NO: 19) (Figure 2). In contrast to dicot sequences of the SQUA clade, monocot sequences of the SQUA clade do not possess a farnesylation motif at their C-terminus.

OsMADS18 dusters up with the corn ZMM28 polypeptide and the barley m3 polypeptide (Becker and Thiessen (2003) Mol Phylogenet Evol 29(3): 464-89). All three proteins comprise the LPPWMLRT amino acid motif within about the last 15 amino acids of their C-terminus. Two other rice MADS box proteins from the SQUA clade, OsMADS14 and OsMADS15, belong to the subclade with the LPPWMLSH motif within about the last 15 amino acids of their C-terminus. For both of these motifs, LPPWMLRT (SEQ ID NO: 18) and LPPWMLSH (SEQ ID NO: 19), the most conserved amino acids are located in the second (P for proline) and fourth (W for tryptophan) positions.

OsMADS18 is a widely expressed gene and its RNA can be detected in root, leaf, inflorescence and developing kernel tissue (Masiero et al. (2002) J Biol Chem 277(29): 26429-35). OsMADS18 may be involved in the transition from vegetative growth to flowering (Fornara et al. (2004) Plant Physiol 135(4): 2207-19). Constitutive overexpression of OsMADS18 in rice leads to dwarfed plants and earlier flowering as a consequence of accelerated plant maturation.

In yeast two hybrid experiments, OsMADS18 has been shown to form heterodimers with OsMADS6, OsMADS24, OsMADS45 and OsMADS47. It also specifically interacts with OsNF-YB1, which shares the highest sequence identity with *Arabidopsis* Leafy Cotyledon1 (LEC1 or AtNF-YB9). Ternary complex formation between the three proteins OsMADS18, OsMADS6 and OsNF-YB1 has also been shown (Masiero et al. (2002) J Biol Chem 277(29): 26429-35).

In US 6,229,068 B1, Yanofsky *et al.* describe a method to increase seed (or fruit) size in a plant by using an AGL8-related gene product and ectopically expressing it in the plant. Preferred regulatory elements mentioned in the document for the ectopic expression of an AGL8-related gene product are constitutive, seed-preferred or inducible elements.

International patent application WO 02/33091 discloses a monocot (from perennial ryegrass) MADS-box transcription factor for the manipulation of flowering and plant architecture. International patent application WO 03/057877 discloses a MADS-box cDNA having a single nucleotide polymorphism (SNP) among different barley varieties.

According to one embodiment of the present invention, there is provided a method for increasing plant seed yield, comprising increasing activity by over expression in a plant of an OsMADS18-like polypeptide or a homologue thereof, which OsMADS18-like polypeptide or homologue thereof is: (i) a monocot type II MADS-box transcription factor of the SQUAMOSA clade; and (ii) has DNA and protein binding activity; and (iii) comprises the motif LPPWMLRT (SEQ ID NO: 18) in the last 15 amino acids of the C terminus of the protein, allowing for one amino acid substitution anywhere in the motif but not in the second and fourth positions of the motif, which are respectively a proline and a tryptophan; and (iv) has at least 65% sequence identity to the OsMADS18 protein of SEQ ID NO: 2.

Advantageously, performance of the methods according to the present invention results in plants having increased yield, particularly seed yield.

The term "increased yield" as defined herein is taken to mean an increase in any one or more of the following, each relative to corresponding wild type plants: (i) increased biomass (weight) of one or more parts of a plant, particularly aboveground (harvestable) parts or increased biomass of any other harvestable part; (ii) increased seed yield, which includes an increase in seed biomass (seed weight) and which may be an increase in the seed weight per plant or on an individual seed basis or an increase in seed weight per hectare or acre; (iii) increased number of flowers (florets) per panicle, which is expressed as a ratio of the number of filled seeds over the number of primary panicles; (iv) increased number of (filled) seeds; (v) increased fill rate of seeds (which is the number of filled seeds divided by the total number of seeds and multiplied by 100); (vi) increased seed size, which may also influence the composition of seeds; (vii) increased seed volume, which may also influence the composition of seeds (for example due to an increase in amount or a change in the composition of oil, protein or carbohydrate); (viii) increased seed area; (ix) increased seed length; (x) increased seed width; (x) increased seed perimeter; (xi) increased harvest index, which is expressed as a ratio of the yield of harvestable parts, such as seeds, over the total biomass; and (xii) increased thousand kernel weight (TKW), which is extrapolated from the number of filled seeds counted and their total weight. An increased TKW may result from an increased seed size and/or seed weight and may also result from an increase in embryo size and/or endosperm size.

Taking corn as an example, a yield increase may be manifested as one or more of the following: increase in the number of plants per hectare or acre, an increase in the number of ears per plant, an increase in the number of rows, number of kernels per row, kernel weight, thousand kernel weight, ear length/diameter, among others. Taking rice as an example, a yield increase may be manifested by an increase in one or more of the following: number of plants per hectare or acre, number of panicles per plant, number of spikelets per panicle, number of flowers per panicle, increase in the seed filling rate, increase in thousand kernel weight, among others. An increase in yield may also result in modified architecture, or may occur as a result of modified architecture.

According to a preferred feature, performance of the methods of the invention result in plants having increased seed yield. Therefore, according to the present invention, there is provided a method for increasing plant seed yield, which method comprises increasing activity in a plant of an OsMADS18-like polypeptide or a homologue thereof.

Since the transgenic plants according to the present invention have increased yield, it is likely that these plants exhibit an increased growth rate (during at least part of their life cycle), relative to the growth rate of corresponding wild type plants at a corresponding stage in their life cycle. The increased growth rate may be specific to one or more parts of a plant (including seeds), or may be throughout substantially the whole plant. A plant having an increased growth rate may even exhibit early flowering. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible. If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of rice plants followed by, for example, the sowing and optional harvesting of soy bean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods disclosed gives plants having an increased growth rate. Therefore, according to the present disclosure, there is provided a method for increasing yield in plants, which method comprises increasing activity in a plant of an OsMADS18-like polypeptide or a homologue thereof.

An increase in yield and/or growth rate occurs whether the plant is under non-stress conditions or whether the plant is exposed to various stresses compared to control/wild type plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the typical stresses to which a plant may be exposed. These stresses may be everyday biotic and/or abiotic (environmental) stresses. Typical abiotic or environmental stresses include temperature stresses caused by atypical hot or cold/freezing temperatures; salt stress; water stress (drought or excess water). Chemicals may also cause abiotic stresses. Biotic stresses are typically those stresses caused by pathogens, such as bacteria, viruses, fungi and insects.

The abovementioned increases in yield may advantageously be obtained in any plant upon performance of the methods of the invention.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen, and microspores, again wherein each of the aforementioned comprise the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising Acacia spp.,Acer spp., *Actinidia* spp., *Aesculus* spp., *Agathis australis, Albizia amara, Alsophila tricolor, Andropogon* spp., *Arachis* spp, Areca *catechu, Astelia fragrans, Astragalus cicer, Baikiaea plurijuga, Betula* spp., *Brassica* spp., *Bruguiera gymnorrhiza, Burkea africana, Butea frondosa, Cadaba farinosa, Calliandra* spp, *Camellia sinensis, Canna indica, Capsicum* spp., *Cassia* spp., *Centroema pubescens, Chaenomeles* spp., *Cinnamomum cassia, Coffea arabica, Colophospermum mopane, Coronillia varia,* Cotoneaster *serotina,* Crataegus spp., *Cucumis* spp., *Cupressus* spp., Cyathea *dealbata, Cydonia oblonga, Cryptomeria japonica, Cymbopogon* spp., *Cynthea dealbata, Cydonia oblonga*, *Dalbergia monetaria, Davallia divaricata, Desmodium* spp., *Dicksonia squarosa, Diheteropogon amplectens, Dioclea* spp, *Dolichos* spp., *Dorycnium rectum, Echinochloa pyramidalis, Ehrartia* spp., *Eleusine* coracana, *Eragrestis* spp., *Erythrina* spp., *Eucalyptus* spp., *Euclea schimperi, Eulalia villosa, Fagopyrum* spp., *Feijoa sellowiana, Fragaria* spp., *Flemingia* spp, *Freycinetia banksii, Geranium thunbergii, Ginkgo biloba*, *Glycine javanica, Gliricidia* spp, *Gossypium hirsutum, Grevillea* spp., *Guibourtia coleosperma, Hedysarum* spp., *Hemarthia altissima,* Heteropogon *contortus, Hordeum vulgare, Hyparrhenia rufa, Hypericum erectum, Hyperthelia dissoluta, Indigo incamata, Iris* spp., *Leptarrhena pyrolifolia, Lespediza* spp., *Lettuca* spp., Leucaena leucocephala, *Loudetia simplex, Lotonus bainesii, Lotus* spp., *Macrotyloma axillare, Malus* spp., *Manihot esculenta, Medicago sativa, Metasequoia glyptostroboides,* Musa *sapientum, Nicotianum* spp., *Onobrychis* spp., *Ornithopus* spp., *Oryza* spp., *Peltophorum africanum, Pennisetum* spp., Persea *gratissima, Petunia* spp., Phaseolus spp., *Phoenix canariensis, Phormium cookianum, Photinia* spp., Picea *glauca, Pinus* spp., *Pisum sativum, Podocarpus totara, Pogonarthria fleckii, Pogonarthria* squarrosa, *Populus* spp., Prosopis *cineraria, Pseudotsuga menziesii, Pterolobium stellatum, Pyrus communis,* Quercus spp., *Rhaphiolepsis umbellata, Rhopalostylis sapida, Rhus natalensis,* Ribes *grossularia,* Ribes spp., *Robinia pseu-*

*doacacia,* Rosa spp., Rubus spp., *Salix* spp., *Schyzachyrium sanguineum, Sciadopitys verticillata, Sequoia sempervirens, Sequoiadendron giganteum, Sorghum bicolor, Spinacia* spp., Sporobolus *fimbriatus, Stiburus alopecuroides,* Stylosanthos *humilis, Tadehagi* spp, *Taxodium distichum, Themeda triandra, Trifolium* spp., *Triticum* spp., *Tsuga heterophylla, Vaccinium* spp., *Vicia* spp., *Vitis vinifera, Watsonia pyramidata, Zantedeschia aethiopica,* Zea *mays,* amaranth, artichoke, asparagus, broccoli, Brussels sprouts, cabbage, canola, carrot, cauliflower, celery, collard greens, flax, kale, lentil, oilseed rape, okra, onion, potato, rice, soybean, strawberry, sugar beet, sugarcane, sunflower, tomato, squash, tea and algae, amongst others. According to a preferred embodiment of the present invention, the plant is a crop plant such as soybean, sunflower, canola, alfalfa, rapeseed, cotton, tomato, potato or tobacco. Further preferably, the plant is a monocotyledonous plant, such as sugar cane. More preferably the plant is a cereal, such as rice, maize, wheat, barley, millet, rye, sorghum or oats.

The activity of an OsMADS18-like polypeptide may be increased by raising levels of the polypeptide. OsMADS18 mRNA levels may also be increased. Alternatively, activity may also be increased when there is no change in levels of an OsMADS18-like polypeptide, or even when there is a reduction in levels of an OsMADS18-like polypeptide. This may occur when the intrinsic properties of the polypeptide are altered, for example, by making mutant versions that are more active that the wild type polypeptide.

The term "OsMADS18-like polypeptide or a homologue thereof" as defined herein refers to a polypeptide (i) being a monocot type II MADS-box transcription factor of the SQUAMOSA clade; and (ii) having DNA and protein binding activity; and (iii) comprising the motif LPPWMLRT (SEQ ID NO: 18) in the last 15 amino acids of the C terminus of the protein, allowing for one amino acid substitution anywhere in the motif but not in the second and fourth positions of the motif, which are respectively a proline and a tryptophan; and (iv) having at least 65% sequence identity to the OsMADS18 protein of SEQ ID NO: 2. The substitution in the motif may be a conservative substitution but is not limited to conservative substitutions. The conservative amino acid substitution may replace any one or more of the amino acid residues of the aforementioned motif, including at positions 2 and 4 as occupied by proline and tryptophan respectively. Conservative substitution tables are readily available in the art. The table below gives examples of conserved amino acid substitutions.

**Table 1: Examples of conserved amino acid substitutions**

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

An "OsMADS18-like polypeptide or a homologue thereof" may readily be identified using routine techniques well known in the art. For example, DNA-binding activity and protein-protein interactions may readily be determined *in vitro* or *in vivo* using techniques well known in the art. Examples of *in vitro* assays for DNA binding activity include: gel retardation analysis using known MADS-box DNA binding domains (West et al. (1998) Nucl Acid Res 26(23): 5277-87), or yeast one-hybrid assays. An example of an *in vitro* assay for protein-protein interactions is the yeast two-hybrid analysis (Fields and Song (1989) Nature 340:245-6).

Furthermore, the motif defined above (SEQ ID NO: 18) may also be readily identified by a person skilled in the art simply by making an alignment and searching for the motif at the C-terminal end of a polypeptide. Similarly, a polypeptide having at least 65% identity to the amino acid sequence represented by SEQ ID NO: 2 may also readily be established by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. Homologues of OsMADS18 comprising the motif LPPWMLRT (SEQ ID NO: 18) within the last 15 amino acids of the protein, and having at least 65% identity to the amino acid sequences represented by SEQ ID NO: 2 may readily be identified using, for example, the ClustalW multiple sequence alignment algorithm (version 1.83) available at http://clustalw.genome.jp/sit-bin/nph-clustalw, with the following pairwise alignment parameters: K-tuple (word) size of 1, window size of 5, gap penalty of 4, number of top diagonal of 5, and a scoring method in percentage.

Examples of plant-derived polypeptides covered by the term "OsMADS18-like polypeptide or a homologue thereof" include (see also Table 2): OsMADS18 AF091458 from *Oryza sativa* (SEQ ID NO: 2), ZMM28 (or m28) AJ430695 from Zea *mays* (SEQ ID NO: 4), MADS3 AY198328 from *Lolium perenne* (SEQ ID NO: 6), m3 AJ249143 from *Hordeum vulgare* (SEQ ID NO: 8). A ZMM28-like protein was deduced from a contig of several *Saccharum officinarum* overlapping ESTs, CA285442 (SEQ ID NO: 9), CA200888 (SEQ ID NO: 10), CA247266 (SEQ ID NO: 11), CA282968 (SEQ ID NO: 12), and CA299090 (SEQ ID NO: 13). The *Saccharum officinarum* protein (SEQ ID NO: 15) was deduced from the consensus sequence (SEQ ID NO: 14) obtained by aligning five different ESTs. SEQ ID NO: 16 is a second nucleotide consensus sequence with one nucleotide change (G to T) at position 578 bp from the ATG, compared to SEQ ID NO: 14. The polypeptide SEQ ID NO: 17 was deduced from SEQ ID NO: 16, and is identical to SEQ ID NO: 15 except for one amino acid change at position 193 of the protein (V193G).

**Table 2: Examples of OsMADS18-like monocot orthologues**

| | Gene name | Accession number | DNA SEQ ID N° | Protein SEQ ID NO | Source |
|---|---|---|---|---|---|
| 1 | OsMADS18 | AF091458 | 1 | 2 | *Oryza sativa* |
| 2 | ZMM28 | AJ430695 | 3 | 4 | *Zea mays* |
| 3 | MADS3 | AY198328 | 5 | 6 | *Lolium perenne* |
| 4 | m3 | AJ249143 | 7 | 8 | *Hordeum vulgare* |
| 5 | ZMM28-like | CA285442<br>CA200888<br>CA247266<br>CA282968<br>CA299090 | 14, 16 (predicted) | 15,17 (predicted) | *Saccharum officinarum* |

It is to be understood that sequences falling under the definition of "OsMADS18-like polypeptide or homologue thereof" are not to be limited to the sequences represented by SEQ ID NO: 2, SEQ ID NO: 4, SEQ ID NO: 6, SEQ ID NO: 8, SEQ ID NO: 15 or SEQ ID NO: 17, but that any polypeptide meeting the criteria of: (i) being a monocot type II MADS-box transcription factor of the SQUAMOSA clade; and (ii) having DNA and protein binding activity; and (iii) comprising the motif LPPWMLRT (SEQ ID NO: 18) in the last 15 amino acids at the C terminus of the protein, allowing for one amino acid substitution anywhere in the motif but not in the second and fourth positions of the motif, which are respectively a proline and a tryptophan; and (iv) having at least 65% sequence identity to the OsMADS18 protein of SEQ ID NO: 2 may be suitable for use in the methods of the invention and to obtain plants having increased yield relative to corresponding wild type plants.

The nucleic acid encoding an OsMADS18-like polypeptide or a homologue thereof may be any natural or synthetic nucleic acid. Therefore the term "OsMADS18-like nucleic acid/gene" as defined herein is any nucleic acid/gene encoding an OsMADS18-like polypeptide or a homologue thereof as defined hereinabove. Examples of OsMADS18-like nucleic acids include those represented by any one of SEQ ID NO: 1, SEQ ID NO: 3, SEQ ID NO: 5, SEQ ID NO: 7, SEQ ID NO: 14 and SEQ ID NO: 16. OsMADS18-like nucleic acids/genes and variants thereof may be suitable in practising the methods of the invention. Variant OsMADS18-like nucleic acid/genes include portions of an OsMADS18-like nucleic acid/gene and/or nucleic acids capable of hybridising with an OsMADS18-like nucleic acid/gene.

The term portion as defined herein refers to a piece of DNA (an OsMADS18-like nucleic acid/gene) comprising at least 747 nucleotides which portion encodes a polypeptide of at least 249 amino acids, which polypeptide comprises at least features (i) and (ii) as follow, preferably together with feature (iii) and/or feature (iv), features (i) to (iv) being: (i) a monocot type II MADS-box transcription factor of the SQUAMOSA clade; (ii) having DNA and protein binding activity; and (iii) comprising the motif LPPWMLRT (SEQ ID NO: 18) in the last 15 amino acids at the C terminus of the protein, allowing for one amino acid substitution anywhere in the motif but not in the second and fourth positions of the motif, which are respectively a proline and a tryptophan; and (iv) having at least 65% sequence identity to the OsMADS18 protein of SEQ ID NO: 2. A portion may be prepared, for example, by making one or more deletions to an OsMADS18-like nucleic acid. The portions may be used in isolated form or they may be fused to other coding (or non coding) sequences in order to, for example, produce a protein that combines several activities. When fused to other coding sequences, the resulting polypeptide produced upon translation may be bigger than that predicted for the OsMADS18-like fragment. Preferably, the functional portion is a portion of a nucleic acid as represented by any one of SEQ ID NO: 1, SEQ ID NO: 3, SEQ ID NO: 5, SEQ ID NO: 7, SEQ ID NO: 14 and SEQ ID NO: 16.

Another variant of an OsMADS18-like nucleic acid/gene is a nucleic acid capable of hybridising under reduced stringency conditions, preferably under stringent conditions, with an OsMADS18-like nucleic acid/gene as hereinbefore defined, which hybridising sequence encodes a polypeptide comprising at least features (i) and (ii) as follow, preferably together with feature (iii) and/or feature (iv), features (i) to (iv) being: (i) a monocot type II MADS-box transcription factor of the SQUAMOSA clade; and (ii) a polypeptide having DNA and protein binding activity; and (iii) a polypeptide comprising the motif LPPWMLRT (SEQ ID NO: 18) located in the last 15 amino acids of the protein, allowing for one amino acid substitution anywhere in the motif but not in the second and fourth positions of the motif, which are respectively a proline and a tryptophan; and (iv) having at least 65% sequence identity to the OsMADS18 protein of SEQ ID NO: 2. In addition to having the aforementioned features, preferably the hybridising sequence is one that is capable of hybridising to a nucleic acid as represented by any one of SEQ ID NO: 1, SEQ ID NO: 3, SEQ ID NO: 5, SEQ ID NO: 7, SEQ ID NO: 14, and SEQ ID NO: 16, or to a portion of any of the aforementioned sequences as defined hereinabove.

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nuclei acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration, ionic strength and hybridisation buffer composition.

"Stringent hybridisation conditions" and "stringent hybridisation wash conditions" in the context of nucleic acid hybridisation experiments such as Southern and Northern hybridisations are sequence dependent and differ under different environmental parameters. The skilled artisan is aware of various parameters which may be altered during hybridisation and washing to either maintain or change the stringency conditions.

The $T_m$ is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M. Formamide reduces the melting temperature of DNA-DNA and DNA-RNA duplexes with 0.6 to 0.7°C for each percent formamide, and addition of 50% formamide allows hybridisaton to be performed at 30 to 45°C, though the rate of hybridisation will be lowered. Base pair mismatches reduce the hybridisation rate and the thermal stability of the duplexes. On average and for large probes, the $T_m$ decreases about 1°C per % base mismatch. The $T_m$ may be calculated using the following equations, depending on the types of hybrids:

1. DNA-DNA hybrids (Meinkoth and Wahl, Anal. Biochem., 138: 267-284, 1984):

$$T_m = 81.5°C + 16.6 x log[Na^+]^a + 0.41 x \%[G/C^b] - 500 x [L^c]^{-1} - 0.61 x \% \text{ formamide}$$

2. DNA-RNA or RNA-RNA hybrids:

$$T_m = 79.8 + 18.5 (\log_{10}[Na^+]^a) + 0.58 (\%G/C^b) + 11.8 (\%G/C^b)^2 - 820/L^c$$

3. oligo-DNA or oligo-RNA[d] hybrids:

For <20 nucleotides: $T_m = 2 (l_n)$
For 20-35 nucleotides: $T_m = 22 + 1.46 (l_n)$

[a] or for other monovalent cation, but only accurate in the 0.01-0.4 M range.
[b] only accurate for %GC in the 30% to 75% range.

[c] L = length of duplex in base pairs.
[d] Oligo, oligonucleotide; $l_n$, effective length of primer = 2×(no. of G/C)+(no. of A/T).

*Note:* for each 1% formamide, the $T_m$ is reduced by about 0.6 to 0.7°C, while the presence of 6 M urea reduces the $T_m$ by about 30°C.

Specificity of hybridisation is typically the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. Generally, suitable stringent conditions for nucleic acid hybridisation assays or gene amplification detection procedures are as set forth above. Conditions of greater or less stringency may also be selected. Generally, low stringency conditions are selected to be about 50°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. For example, stringent conditions are those that are at least as stringent as, for example, conditions A-L; and reduced stringency conditions are at least as stringent as, for example, conditions M-R. Non-specific binding may be controlled using any one of a number of known techniques such as, for example, blocking the membrane with protein containing solutions, additions of heterologous RNA, DNA, and SDS to the hybridisation buffer, and treatment with RNase. Examples of hybridisation and wash conditions are listed in Table 3 below.

**Table 3: Examples of hybridisation and wash conditions**

| Stringency Condition | Polynucleotide Hybrid ± | Hybrid Length (bp) ‡ | Hybridization Temperature and Buffer † | Wash Temperature and Buffer† |
|---|---|---|---|---|
| A | DNA: DNA | > or equal to 50 | 65°C 1×SSC; or 42°C, 1×SSC and 50% formamide | 65°C; 0.3×SSC |
| B | DNA:DNA | <50 | Tb*; 1×SSC | Tb*; 1×SSC |
| C | DNA:RNA | > or equal to 50 | 67°C 1×SSC; or 45°C, 1×SSC and 50% formamide | 67°C; 0.3×SSC |
| D | DNA:RNA | <50 | Td*; 1×SSC | Td*; 1×SSC |
| E | RNA:RNA | > or equal to 50 | 70°C 1×SSC; or 50°C, 1×SSC and 50% formamide | 70°C; 0.3×SSC |
| F | RNA:RNA | <50 | Tf*; 1×SSC | Tf*; 1×SSC |
| G | DNA:DNA | > or equal to 50 | 65°C 4xSSC; or 45°C, 4xSSC and 50% formamide | 65°C; 1×SSC |
| H | DNA: DNA | <50 | Th*; 4 ×SSC | Th*; 4×SSC |
| I | DNA:RNA | > or equal to 50 | 67°C 4xSSC; or 45°C, 4xSSC and 50% formamide | 67°C; 1×SSC |
| J | DNA:RNA | <50 | Tj*; 4 ×SSC | Tj*; 4 ×SSC |
| K | RNA:RNA | > or equal to 50 | 70°C 4×SSC; or 40°C, 6xSSC and 50% formamide | 67°C; 1×SSC |
| L | RNA:RNA | <50 | Tl*; 2 ×SSC | Tl*; 2xSSC |
| M | DNA: DNA | > or equal to 50 | 50°C 4xSSC; or 40°C, 6×SSC and 50% formamide | 50°C; 2×SSC |

(continued)

| Stringency Condition | Polynucleotide Hybrid ± | Hybrid Length (bp) ‡ | Hybridization Temperature and Buffer † | Wash Temperature and Buffer† |
|---|---|---|---|---|
| N | DNA:DNA | <50 | Tn*; 6 ×SSC | Tn*; 6xSSC |
| O | DNA:RNA | > or equal to 50 | 55°C 4xSSC; or 42°C, 6xSSC and 50% formamide | 55°C; 2×SSC |
| P | DNA:RNA | <50 | Tp*; 6 ×SSC | Tp*; 6×SSC |
| Q | RNA:RNA | > or equal to 50 | 60°C 4xSSC; or 45°C, 6xSSC and 50% formamide | 60°C.; 2xSSC |
| R | RNA:RNA | <50 | Tr*; 4 ×SSC | Tr*; 4xSSC |

‡ The "hybrid length" is the anticipated length for the hybridising nucleic acid. When nucleic acids of known sequence are hybridised, the hybrid length may be determined by aligning the sequences and identifying the conserved regions described herein.
† SSPE (1×SSPE is 0.15M NaCl, 10mM $NaH_2PO_4$, and 1.25mM EDTA, pH7.4) may be substituted for SSC (1×SSC is 0.15M NaCl and 15mM sodium citrate) in the hybridisation and wash buffers; washes are performed for 15 minutes after hybridisation is complete. The hybridisations and washes may additionally include 5 × Denhardt's reagent, 0.5-1.0% SDS, 100 μg/ml denatured, fragmented salmon sperm DNA, 0.5% sodium pyrophosphate, and up to 50% formamide.
* Tb-Tr: The hybridisation temperature for hybrids anticipated to be less than 50 base pairs in length should be 5-10°C less than the melting temperature $T_m$ of the hybrids; the $T_m$ is determined according to the abovementioned equations.
± The present invention also encompasses the substitution of any one, or more DNA or RNA hybrid partners with either a PNA, or a modified nucleic acid.

For the purposes of defining the level of stringency, reference can be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989).

The OsMADS18-like nucleic acid or variant thereof may be derived from any natural or artificial source. The nucleic acid/gene or variant thereof may be isolated from a microbial source, such as yeast or fungi, or from a plant, algae or animal (including human) source. This nucleic acid may be modified from its native form in composition and/or genomic environment through deliberate human manipulation. The nucleic acid is preferably of plant origin, whether from the same plant species (for example to the one in which it is to be introduced) or whether from a different plant species. The nucleic acid may be isolated from a monocotyledonous species, preferably from the family Poaceae, further preferably from *Oryza sativa.* More preferably, the OsMADS18-like nucleic acid isolated from *Oryza sativa* is represented by SEQ ID NO: 1 and the OsMADS18-like amino acid sequence is as represented by SEQ ID NO: 2.

The activity of an OsMADS18-like polypeptide or a homologue thereof may be increased by introducing a genetic modification (preferably in the locus of an OsMADS18-like gene). The locus of a gene as defined herein is taken to mean a genomic region, which includes the gene of interest and 10 kb up- or down stream of the coding region.

The genetic modification may be introduced, for example, by any one (or more) of the following methods: T-DNA activation, TILLING, site-directed mutagenesis, directed evolution, homologous recombination or by introducing and expressing in a plant a nucleic acid encoding an OsMADS18-like polypeptide or a homologue thereof. Following introduction of the genetic modification, there follows a step of selecting for increased activity of an OsMADS18-like polypeptide, which increase in activity gives plants having increased yield.

T-DNA activation tagging (Hayashi et al. Science (1992) 1350-1353) involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to overexpression of genes near the inserted T-DNA. The resulting transgenic plants show dominant phenotypes due to overexpression of genes close to the introduced promoter. The promoter to be introduced may be any promoter capable of directing expression of a gene in the desired organism, in this case a plant. For example, constitutive, tissue-preferred, cell type-preferred and inducible promoters are all suitable for use in T-DNA activation.

A genetic modification may also be introduced in the locus of an OsMADS18-like gene using the technique of TILLING (Targeted Induced Local Lesions In Genomes). This mutagenesis technology is useful to generate, identify and isolate mutagenised variants of an OsMADS18-like nucleic acid capable of exhibiting OsMADS18-like activity. TILLING also allows selection of plants carrying such mutant variants. These mutant variants may even exhibit higher OsMADS18-like activity than that exhibited by the gene in its natural form. TILLNG combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Site-directed mutagenesis may be used to generate variants of OsMADS18-like nucleic acids. Several methods are available to achieve site-directed mutagenesis, the most common being PCR based methods (current protocols in molecular biology. Wiley Eds. http://www.4ulr.com/products/currentprotocols/index.html).

Directed evolution may also be used to generate variants of OsMADS18-like nucleic acids. This consists of iterations of DNA shuffling followed by appropriate screening and/or selection to generate variants of OsMADS18-like nucleic acids or variants thereof encoding OsMADS18-like polypeptides or homologues thereof having a modified biological activity (Castle et al., (2004) Science 304(5674): 1151-4; US patents 5,811,238 and 6,395,547).

T-DNA activation, TILLING, site-directed mutagenesis and directed evolution are examples of technologies that enable the generation of novel alleles and OsMADS18-like variants.

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2):132-8). The nucleic acid to be targeted (which may be an OsMADS18-like nucleic acid or variant thereof as hereinbefore defined) need not be targeted to the locus of an OsMADS18-like gene, but may be introduced in, for example, regions of high expression. The nucleic acid to be targeted may be an improved allele used to replace the endogenous gene or may be introduced in addition to the endogenous gene.

According to a preferred embodiment of the invention, plant yield may be improved by introducing and expressing in a plant a nucleic acid encoding an OsMADS18-like polypeptide or a homologue thereof.

A preferred method for introducing a genetic modification (which in this case need not be in the locus of an OsMADS18-like gene) is to introduce and express in a plant a nucleic acid encoding an OsMADS18-like polypeptide or a homologue thereof. An OsMADS18-like polypeptide or a homologue thereof as mentioned above is (i) a monocot type II MADS-box transcription factor of the SQUAMOSA clade; and (ii) has DNA and protein binding activity; and (iii) comprises the motif LPPWMLRT (SEQ ID NO: 18) located in the last 15 amino acids of the protein, allowing for one amino acid substitution anywhere in the motif but not in the second and fourth positions of the motif, which are respectively a proline and a tryptophan; and (iv) has at least 65 % sequence identity to the OsMADS18 protein of SEQ ID NO: 2. The nucleic acid to be introduced into a plant may be a full-length nucleic acid or may be a portion or a hybridizing sequence as hereinbefore defined.

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived. To produce such homologues, amino acids of the protein may be replaced by other amino acids having similar properties (such as similar hydrophobicity, hydrophilicity, antigenicity, propensity to form or break $\alpha$-helical structures or $\beta$-sheet structures). Conservative substitution tables are well known in the art (see for example Creighton (1984) Proteins. W.H. Freeman and Company and Table 1 above).

Also encompassed by the term "homologues" are two special forms of homology, which include orthologous sequences and paralogous sequences, which encompass evolutionary concepts used to describe ancestral relationships of genes. The term "paralogous" relates to gene-duplications within the genome of a species leading to paralogous genes. The term "orthologous" relates to homologous genes in different organisms due to speciation.

Orthologues in, for example, other monocot plant species may easily be found by performing a so-called reciprocal blast search. This may be done by a first blast involving blasting a query sequence (for example, SEQ ID NO:

1 or SEQ ID NO: 2) against any sequence database, such as the publicly available NCBI database which may be found at: http://www.ncbi.nlm.nih.gov. BLASTn or tBLASTX (using standard default values) may be used when starting from nucleotide sequence and BLASTP or TBLASTN (using standard default values) may be used when starting from a protein sequence. The BLAST results may optionally be filtered. The sequences of either the filtered or non-filtered results are then BLASTed back (second BLAST) against the sequences from the same organism as the query sequence organism, (where the query sequence is SEQ ID NO: 1 or SEQ ID NO: 2 the second blast would therefore be against *Oryza sativa* (rice) sequences). The results of the first and second BLASTs are then compared. A paralogue is identified if a high-ranking hit from the second blast is from the same species as from which the query sequence is derived; an orthologue is identified if a high-ranking hit is not from the same species as from which the query sequence is derived. High-ranking hits are those having a low E-value. The lower the E-value, the more significant the score (or in other words the lower the chance that the hit was found by chance). Computation of the E-value is well known in the art. In the case of large families, ClustalW may be used, followed by a neighbour joining tree, to help visualize clustering of related genes and to identify orthologues and paralogues.

A homologue may be in the form of a "substitutional variant" of a protein, i.e. where at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues. Preferably, amino acid substitutions comprise conservative amino acid substitutions.

A homologue may also be in the form of an "insertional variant" of a protein, i.e. where one or more amino acid residues are introduced into a predetermined site in a protein. Insertions may comprise amino-terminal and/or carboxy-terminal fusions as well as intra-sequence insertions of single or multiple amino acids. Generally, insertions within the amino acid sequence will be smaller than amino- or carboxy-terminal fusions, of the order of about 1 to 10 residues. Examples of amino- or carboxy-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag·100 epitope, c-myc epitope, FLAG®-epitope, lacZ, CMP (calmodulin-binding peptide), HA epitope, protein C epitope and VSV epitope.

Homologues in the form of "deletion variants" of a protein are characterised by the removal of one or more amino acids from a protein.

Amino acid variants of a protein may readily be made using peptide synthetic techniques well known in the art, such as solid phase peptide synthesis and the like, or by recombinant DNA manipulations. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen *in vitro* mutagenesis (USB, Cleveland, OH), QuickChange Site Directed mutagenesis (Stratagene, San Diego, CA), PCR-mediated site-directed mutagenesis or other site-directed mutagenesis protocols.

The OsMADS18-like polypeptide or homologue thereof may be a derivative. "Derivatives" include peptides, oligopeptides, polypeptides, proteins and enzymes which may comprise substitutions, deletions or additions of naturally and non-naturally occurring amino acid residues compared to the amino acid sequence of a naturally-occurring form of the protein, for example, as presented in SEQ ID NO: 2. "Derivatives" of a protein encompass peptides, oligopeptides, polypeptides, proteins and enzymes which may comprise naturally occurring altered, glycosylated, acylated or non-naturally occurring amino acid residues compared to the amino acid sequence of a naturally-occurring form of the polypeptide. A derivative may also comprise one or more non-amino acid substituents compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein.

The OsMADS18-like polypeptide or homologue thereof may be encoded by an alternative splice variant of an OsMADS18-like nucleic acid/gene. The term "alternative splice variant" as used herein encompasses variants of a nucleic acid sequence in which selected introns and/or exons have been excised, replaced or added, or in which introns have been shortened or lengthened. Such variants will be ones in which the biological activity of the protein is retained, which may be achieved by selectively retaining functional segments of the protein. Such splice variants may be found in nature or may be manmade. Methods for making such splice variants are well known in the art. Preferred splice variants are splice variants of the nucleic acid represented by SEQ ID NO: 1, SEQ ID NO: 3, SEQ ID NO: 5, SEQ ID NO: 7, SEQ ID NO: 14 and SEQ ID NO: 16. Further preferred are splice variants encoding a polypeptide comprising at least features (i) and (ii) as follow, preferably together with feature (iii) and/or feature (iv), features (i) to (iv) being (i) a monocot type II MADS-box transcription factor of the SQUAMOSA clade; and (ii) having DNA and protein binding activity; and (iii) comprising the motif LPPWMLRT (SEQ ID NO: 18) located in the last 15 amino acids of the protein, allowing for one amino acid substitution anywhere in the motif but not except in the second and fourth positions of the motif, which are respectively a proline and a tryptophan; and (iv) having at least 65% sequence identity to the OsMADS18 protein of SEQ ID NO: 2.

The homologue may also be encoded by an allelic variant of a nucleic acid encoding an OsMADS18-like polypeptide or a homologue thereof, preferably an allelic variant of a nucleic acid represented by SEQ ID NO: 1, SEQ ID NO: 3, SEQ ID NO: 5, SEQ ID NO: 7, SEQ ID NO: 14 or SEQ ID NO: 16. Further preferably, the polypeptide encoded by the allelic variant comprises at least features (i) and (ii) as follow, preferably together with feature (iii) and/or feature (iv), features (i) to (iv) being: (i) a monocot type II MADS-box transcription factor of the SQUAMOSA clade; and (ii) having DNA and protein binding activity; and (iii) comprising the motif LPPWMLRT (SEQ ID NO: 18) located in the last 15 amino acids of the protein, allowing for one amino acid substitution anywhere in the motif but not in the second and fourth positions of the motif, which are respectively a proline and a tryptophan; and (iv) having at least 65% sequence identity to the OsMADS18 protein of SEQ ID NO: 2. Allelic variants exist in nature, and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

According to a preferred aspect of the present invention, enhanced or increased expression of the OsMADS18-like nucleic acid or variant thereof is envisaged. Methods for obtaining enhanced or increased expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of an OsMADS18-like nucleic acid or variant thereof. For example, endogenous promoters may be altered *in vivo* by mutation, deletion or substitution (see, Kmiec, U.S. Pat. No. 5,565,350; Zarling et al., PCT/US93/03868), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold, Buchman and Berg, Mol. Cell biol. 8:4395-4405 (1988); Callis et al., Genes Dev. 1:1183-1200 (1987). Such intron enhancement of gene expression is typically greatest when placed near the 5' end of the transcription unit. Use of the maize introns Adh1-S intron 1, 2, and 6, the Bronze-1 intron are known in the art. See generally, The Maize Handbook, Chapter 116, Freeling and Walbot, Eds., Springer, N.Y. (1994).

The invention also provides genetic constructs and vectors to facilitate introduction and/or expression of the nucleotide sequences useful in the methods according to the invention so as to give plants having increased yield relative to corresponding wild type plants.

Therefore, there is provided a gene construct comprising:

(i) An OsMADS18-like nucleic acid or variant thereof;
(ii) One or more control sequences capable of driving expression of the nucleic acid sequence of (i); and optionally
(iii) A transcription termination sequence.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells.

Plants are transformed with a vector comprising the sequence of interest (i.e., an OsMADS18-like nucleic acid or variant thereof). The sequence of interest is operably linked to one or more control sequences (at least to a promoter). The terms "regulatory elements, "control sequence" and "promoter" are all used interchangeably herein and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ. The term "operably linked" as used herein refers to a functional linkage between the promoter sequence and the gene of interest, such that the promoter sequence is able to initiate transcription of the gene of interest.

The OsMADS18-like nucleic acid or variant thereof is preferably operably linked to an early shoot apical meristem promoter. An "early shoot apical meristem promoter" as defined herein is a promoter that is transcriptionally active in the shoot apical meristem from the embryo globular stage up to the young seedling stage; these stages being well known to persons skilled in the art. Preferably, the early shoot apical meristem promoter is an OSH1 promoter (from rice; SEQ ID NO: 22 (Matsuoka et al., (1993) Plant Cell 5: 1039-1048; Sato et al., (1996) Proc Natl Acad Sci U S A 93(15): 8117-22). It should be clear that the applicability of the present invention is not restricted to the OsMADS18-like nucleic acid represented by SEQ ID NO: 1, nor is the applicability of the invention restricted to expression of an OsMADS18-like nucleic acid when driven by an OSH1 promoter. Examples of other early shoot apical meristem promoters are shown in Table 4 below. These are members of the KNOX family class 1 homeobox, from paralogous or orthologous genes. It should be understood that the list below is non-exhaustive.

**Table 4: Examples of early apical meristem promoters**

| Gene source | Gene family | Plant source | Reference |
|---|---|---|---|
| OSH1 | KNOX family class 1 homeobox | *Oryza sativa* | -Matsuoka et al., (1993) Plant Cell 5: 1039-1048 -Sato et al., (1996) PNAS 93: 8117-8122 |
| Knotted 1 | KNOX family class 1 homeobox | *Zea mays* | Hake et al., (1989) EMBO Journal 8: 15-22 |
| KNAT1 | KNOX family class 1 homeobox | *Arabidopsis thaliana* | Lincoln et al., (1994) Plant Cell 6: 1859-1876 |
| Oskn2 | KNOX family class 1 homeobox | *Oryza sativa* | Postma-Haarsma et al., (1999) Plant Mol Biol 39(2): 257-71 |
| Oskn3 | KNOX family class 1 homeobox | *Oryza sativa* | Postma-Haarsma et al., (1999) Plant Mol Biol 39(2): 257-71 |

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences that may be suitable for use in performing the invention. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene that confers a phenotype on a cell in which it is expressed to facilitate the identification and/or selection of cells that are transfected or transformed with a nucleic acid construct of the invention. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin), to herbicides (for example bar which provides resistance to Basta; aroA or gox providing resistance against glyphosate), or genes that provide a metabolic trait (such as manA that allows plants to use mannose as sole carbon source). Visual marker genes result in the formation of colour (for example β-glucuronidase, GUS), luminescence (such as luciferase) or fluorescence (Green Fluorescent Protein, GFP, and derivatives thereof).

The present invention also encompasses plants obtainable by the methods according to the present invention. The present invention therefore provides plants obtainable by the methods according to the present invention, which plants have introduced therein an OsMADS18-like nucleic acid or variant thereof comprised in a construct as defined above.

The invention also provides a method for the production of transgenic plants having increased yield relative to corresponding wild type plants, comprising introduction and expression in a plant of an OsMADS18-like nucleic acid or a variant thereof.

More specifically, the present invention provides a method for the production of transgenic plants having increased yield relative to corresponding wild type plants, which method comprises:

(i) introducing and expressing in a plant, plant part or plant cell an OsMADS18-like nucleic acid or variant thereof; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al., 1985 Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic rice plants expressing an OsMADS18-like nucleic acid/gene are preferably produced via Agrobacferium-mediated transformation using any of the well known methods for rice transformation, such as described in any of the following: published European patent application EP1198985 A1; Aldemita and Hodges (Planta 199: 612-617, 1996); Chan et al. (Plant Mol Biol 22 (3): 491-506, 1993); Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention. The invention also includes host cells containing an isolated OsMADS18-like nucleic acid or variant thereof. Preferred host cells according to the invention are plant cells. The invention also extends to harvestable parts of a plant such as, but not limited to seeds, leaves, fruits, flowers, stem cultures, rhizomes, tubers and bulbs. Products derived from a harvestable part of such a plant, such as dry pellets or powders, oil, fat and fatty acids, starch or proteins are described.

The present invention also encompasses use of OsMADS18-like nucleic acids or variants thereof and use of OsMADS18-like polypeptides or homologues thereof.

One such use relates to improving plant yield relative to corresponding wild type plants, in particular in improving seed yield. The increase in yield being as defined hereinabove.

OsMADS18-like nucleic acids or variants thereof, or OsMADS18-like polypeptides or homologues thereof may find use in breeding programmes in which a DNA marker is identified which may be genetically linked to an OsMADS18-like gene or variant thereof. The OsMADS18-like nucleic acids/ genes or variants thereof, or OsMADS18-like polypeptides or homologues thereof may be used to define a molecular marker. This DNA or protein marker may then be used in breeding programmes to select plants having increased yield. The OsMADS18-like gene or variant thereof may, for example, be a nucleic acid as represented by any one of SEQ ID NO: 1, SEQ ID NO: 3, SEQ ID NO: 5, SEQ ID NO: 7, SEQ ID NO: 14, and SEQ ID NO: 16.

Allelic variants of an OsMADS18-like nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring yield performance of plants containing different allelic variants of the sequence in question, for example, different allelic variants of any one of SEQ ID NO: 1, SEQ ID NO: 3, SEQ ID NO: 5, SEQ ID NO: 7, SEQ ID NO: 14, and SEQ ID NO: 16. The yield performance may be monitored in a greenhouse or in the field. Further optional steps include crossing plants, in which the superior allelic variant was identified, with another plant. This could be used, for example, to make a combination of interesting phenotypic features.

An OsMADS18-like nucleic acid or variant thereof may also be used as probes for genetically and physically mapping the genes that they are a part of, and as markers for traits linked to those genes. Such information may be useful in plant breeding in order to develop lines with desired phenotypes. Such use of OsMADS18-like nucleic acids or variants thereof requires only a nucleic acid sequence of at least 15 nucleotides in length. The OsMADS18-like nudeic acids or variants thereof may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the OsMADS18-like nucleic acids or variants thereof. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the OsMADS18-like nucleic acid or variant thereof in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridization (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield, as described hereinbefore. The trait of increased yield may also be combined with other economically advantageous traits, such as further yield-enhancing traits, tolerance to various stresses, traits modifying various architectural features and/or biochemical and/or physiological features.

**Description of figures**

The present invention will now be described with reference to the following figures in which:

**Fig. 1** shows the typical domain structure of MIKC MADS box transcription factors. The MADS domain is located at the amino-terminal end of the protein and encodes a DNA binding and dimerization function. The conserved K domain is involved in protein dimerisation. The I and the C domains are less well conserved. The C domain can be involved in transcriptional activation or in the formation of higher-order MADS multimers (from Jack (2001) Plant Molec Biol 46: 515-520).

**Fig. 2** shows a multiple alignment of several plant MADS domain transcription factors of the SQUAMOSA clade, using VNTI AlignX multiple alignment program, based on a modified ClustalW algorithm (InforMax, Bethesda, MD, http://www.informaxinc.com), with default settings for gap opening penalty of 10 and a gap extension of 0.05). The SQUAMOSA-specific residues of the MADS domain are boxed across the alignment including the MADS consensus sequence. The two dicot subclades within the SQUAMOSA clade are indicated as dicot AP1 clade and dicot FUL clade. The monocot sequences are further subdivided with respect to their conserved motifs in the C-terminal end of the protein, LPPWMLRT and LPPWMLSH that are boxed in bold.

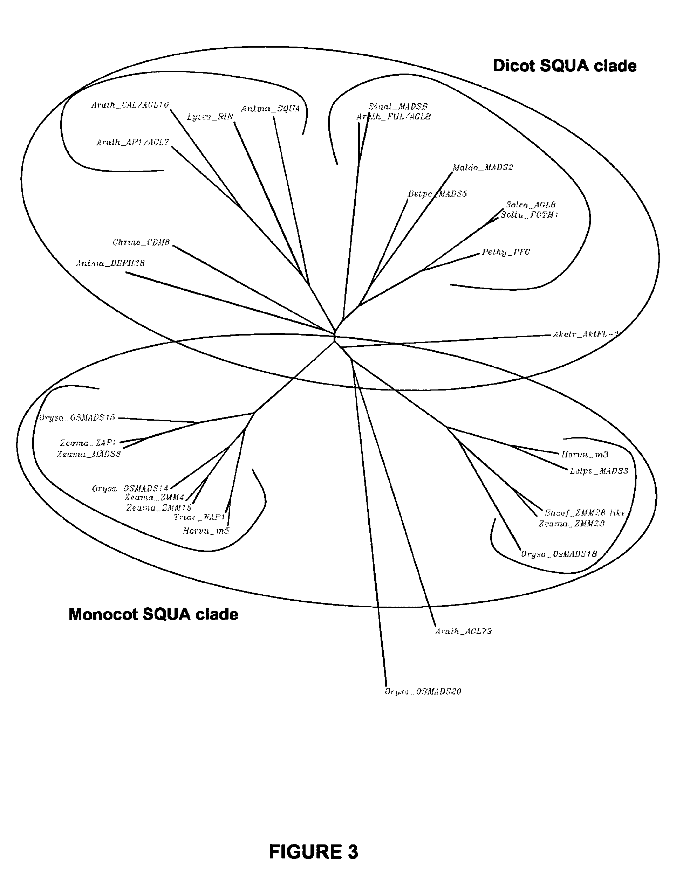

Fig.3 represents an unrooted neighbour joining tree, derived from a sequence alignment using ClustalW 1.83 with default values. The two major groups, comprising monocots and dicots proteins, can each be further split up into AP1 and FUL subclades for the dicots, and C-terminal-comprising motifs LPPWMLRT (SEQ ID NO: 18) and LPPWMLSH (SEQ ID NO: 19) for the monocots. The source, description and accession number of the polypeptide sequences used are given in the table below.

| **Source** | **Description** | **NCBI accession number** |
|---|---|---|
| *Akebia trifoliata* | FRUITFULL-like protein; Aketr_AktFL-1 | AAT46099 |
| *Antirrhinum majus* | DEFH28; Antma_DEFH28 | AAK72467 |
| *Antirrhinum majus* | SQUA; Antma_SQUA | CAA45228 |
| *Arabidopsis thaliana* | MADS-box protein AGL79; Arath_AGL79 | AAN52802 |
| *Arabidopsis thaliana* | APETALA1/AGL7; Arath_AP1/AGL7 | P35631 |
| *Arabidopsis thaliana* | CAL/AGL 10; Arath_CAL/AGL10 | Q39081 |
| *Arabidopsis thaliana* | FUL/AGL8; Arath_FUL/AGL8 | Q38876 |
| *Betula pendula* | MADS5; Betpe_MADS5 | CAA67969 |
| *Chrysanthemum x morifolium* | CDM8; Chrmo_CDM8 | AAO22981 |
| *Hordeum vulgare* | MADS-box protein 3; Horvu_m3 | CAB97351 |
| *Hordeum vulgare* | MADS-box protein 5; Horvu_m5 | CAB97352 |
| *Lolium perenne* | MADS3; Lolpe_MADS3 | AAO45875 |
| *Lycopersicum esculentum* | RIN; Lyces_RIN | AAM15775 |
| *Malus* X *domestica* | MADS2; Maldo_MADS2 | AAC83170 |
| *Oryza sativa* | OsMADS14; Orysa_OsMADS14 | AAF19047 |
| *Oryza sativa* | OsMADS15; Orysa_OsMADS15 | AAF19048 |
| *Oryza sativa* | OsMADS18; Orysa_OsMADS18 | AAF04972 |
| *Oryza sativa* | OsMADS20; Orysa_OsMADS20 | AAO92341 |
| *Petunia hybrida* | PFG; Pethy_PFG | AAQ72509 |

(continued)

| Source | Description | NCBI accession number |
|---|---|---|
| *Saccharum officinarum* | ZMM28-like; Sacof_ZMM28 like | SEQ ID NO:15, 17 |
| *Sinapis alba* | MADS B; Sinal_MADSB | Q41274 |
| *Solanum commersonii* | AGL8 homolog; Solco_AGL8 | 022328 |
| *Solanum tuberosum* | POTM1-1; Soltu_POTM1-1 | Q42429 |
| *Triticum aestivum* | WAP1; Triae_WAP1 | BAA33457 |
| *Zea mays* | MADS3; Zeama_MADS3 | AAG43200 |
| *Zea mays* | ZAP1; Zeama_ZAP1 | AAB00081 |
| *Zea mays* | ZMM15; Zeama_ZMM15 | CAD23408 |
| *Zea mays* | ZMM28; Zeama_ZMM28 | CAD23441 |
| *Zea mays* | ZMM4; Zeama_ZMM4 | CAD23417 |

Fig. 4 shows a binary vector p0643, for expression in *Oryza sativa* of an *Oryza sativa* OsMADS18-like (internal reference CDS2787) under the control of an OSH1 promoter (internal reference PRO0200).

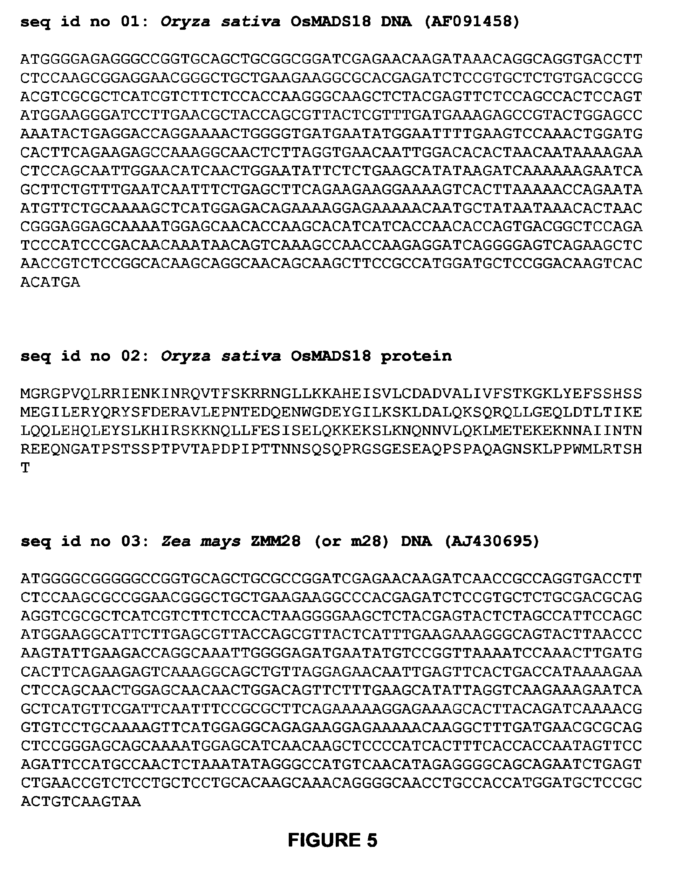

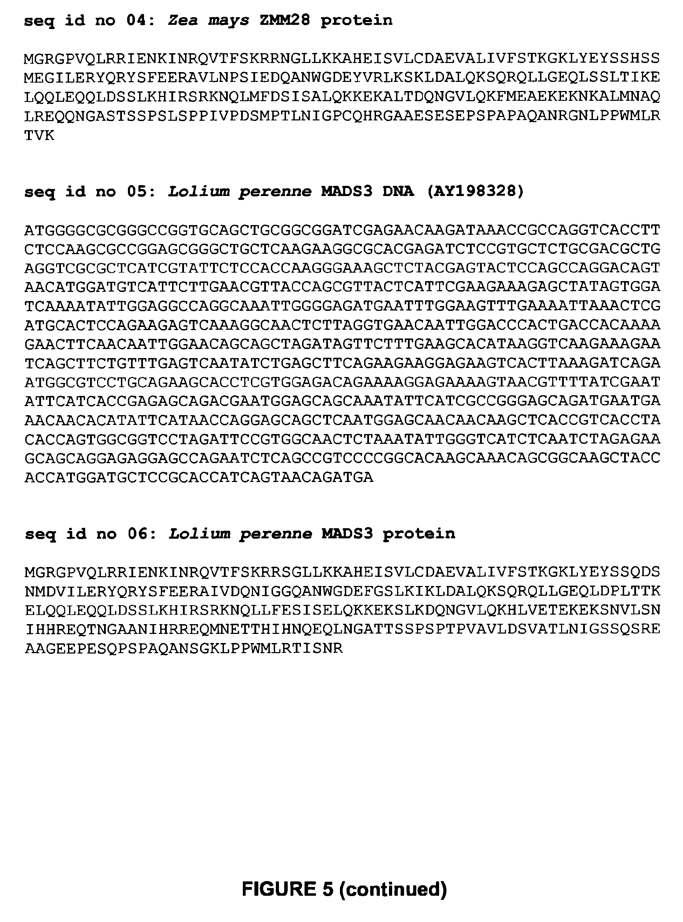

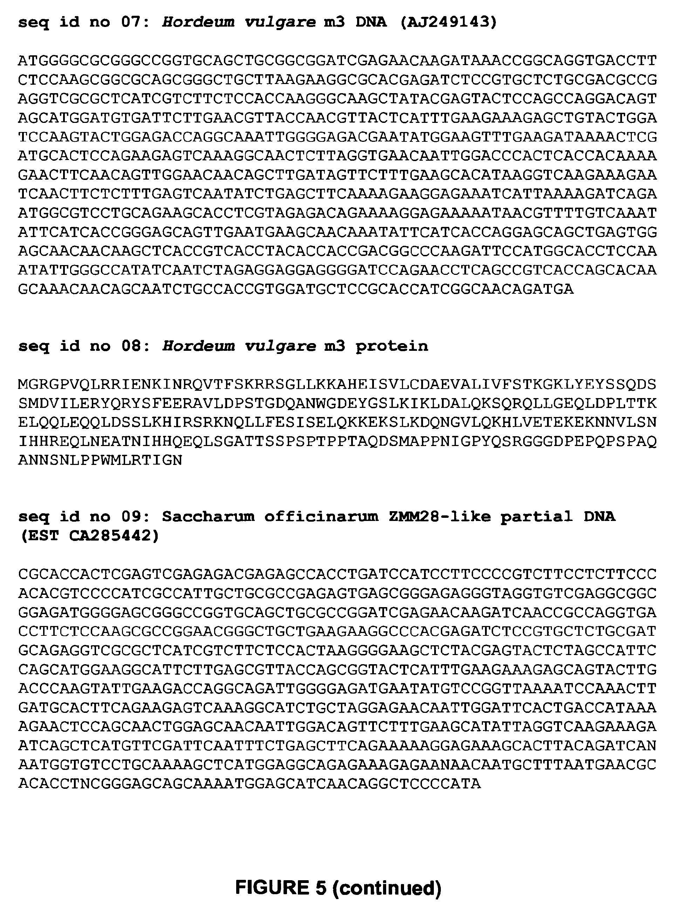

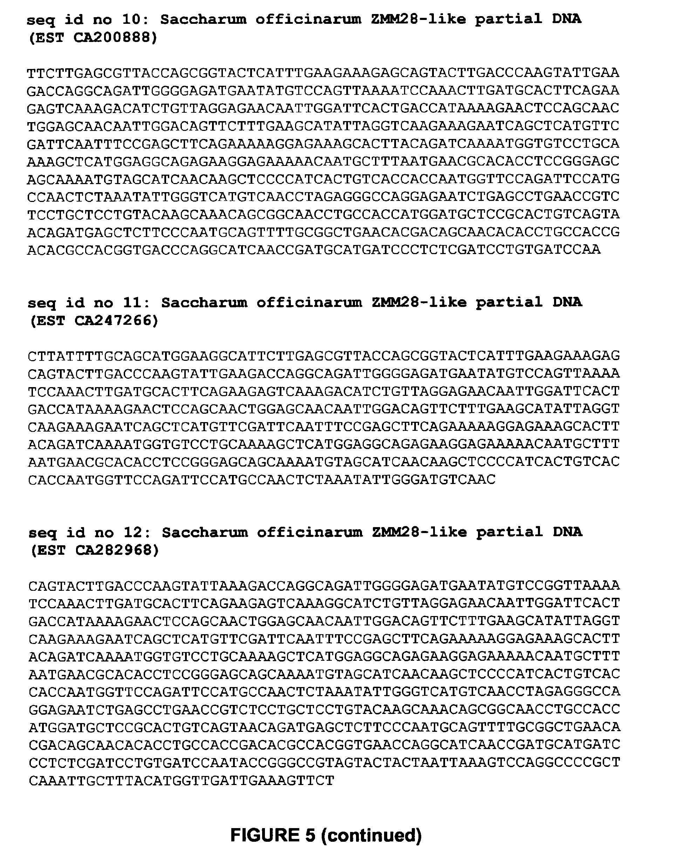

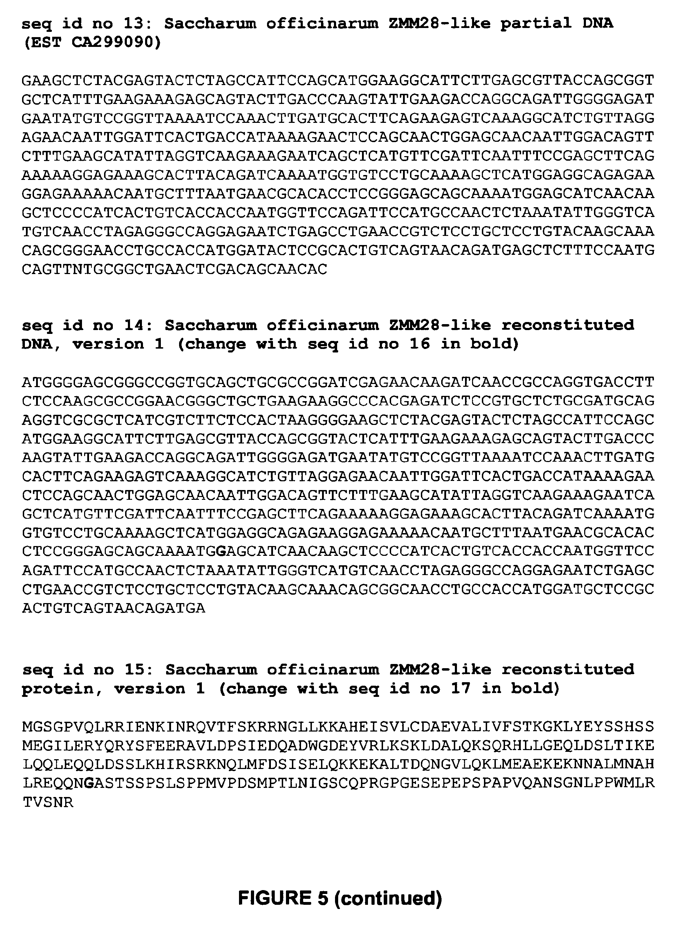

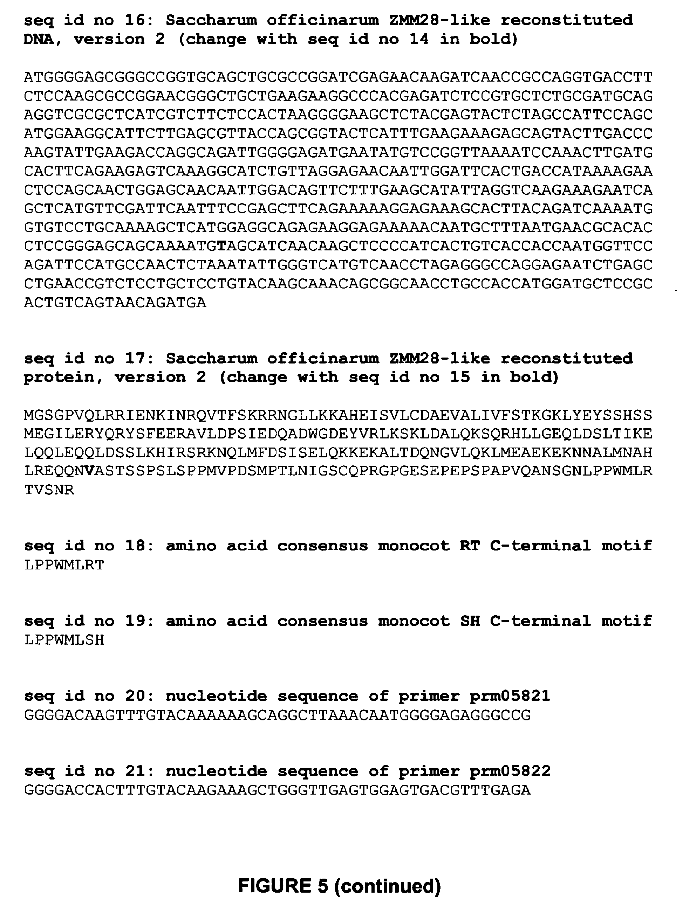

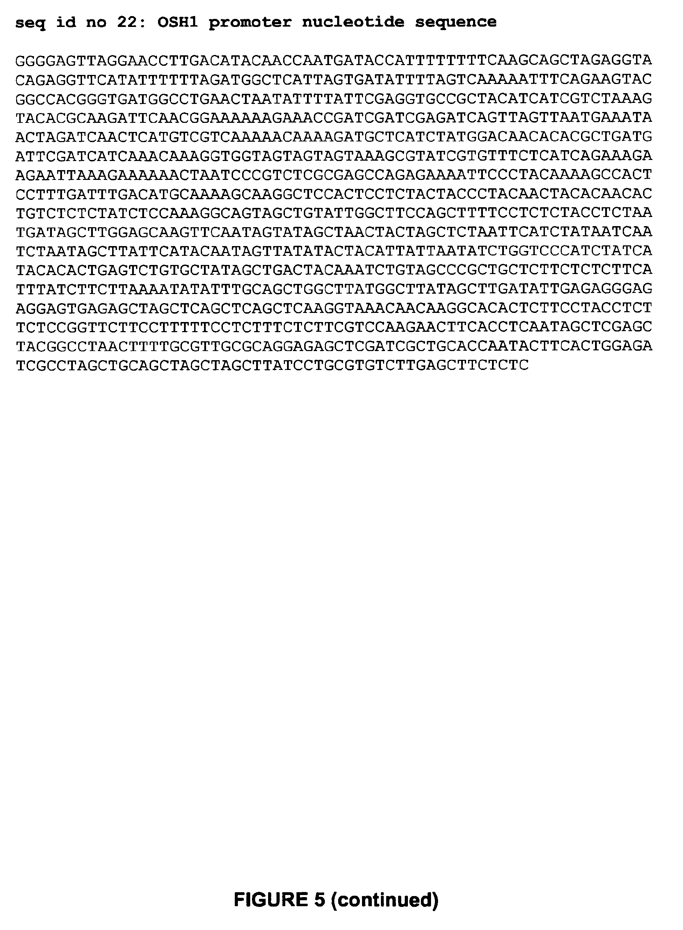

Fig. 5 details examples of sequences useful in performing the methods according to the present invention.

Examples

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

***Example 1: Gene Cloning***

The *Oryza sativa* OsMADS18-like gene (CDS2787) was amplified by PCR using as template an *Oryza sativa* seedling cDNA library (Invitrogen, Paisley, UK). After reverse transcription of RNA extracted from seedlings, the cDNAs were cloned into pCMV Sport 6.0. Average insert size of the bank was 1.6 kb and the original number of clones was of the order of $1.67 \times 10^7$ cfu. Original titer was determined to be $3.34 \times 10^6$ cfu/ml after first amplification of $6 \times 10^{10}$ cfu/ml. After plasmid extraction, 200 ng of template was used in a 50 μl PCR mix. Primers prm05821 (SEQ ID NO: 20; sense, start codon in bold, AttB1 site in italic: 5'-*GGGGACAAGTTTGTACAAAAAAGCAGG*CTTCACA**ATG**GGGAGAGGGCCG 3') and prm05822 (SEQ ID NO: 21; reverse, complementary, AttB2 site in italic: 5' *GGGGACCACTTTGTACAAGAAAGCTGGGT*TGAGTGGAGTGACGTTTGAGA3'), which include the AttB sites for Gateway recombination, were used for PCR amplification. PCR was performed using Hifi Taq DNA polymerase in standard conditions. A PCR fragment of 849 bp (including attB sites) was amplified and purified also using standard methods. The first step of the Gateway procedure, the BP reaction, was then performed, during which the PCR fragment recombines *in vivo* with the pDONR201 plasmid to produce, according to the Gateway terminology, an "entry clone", p05838. Plasmid pDONR201 was purchased from Invitrogen, as part of the Gateway® technology.

***Example 2: Vector Construction***

The entry clone p05838 was subsequently used in an LR reaction with p05294, a destination vector used for *Oryza sativa* transformation. This vector contains as functional elements within the T-DNA borders: a plant selectable marker; a screenable marker expression cassette; and a Gateway cassette intended for LR *in vivo* recombination with the sequence of interest already cloned in the entry clone. A rice OSH1 promoter (SEQ ID NO 22) for early apical meristem expression (PRO0200) was located upstream of this Gateway cassette (Matsuoka et al., Plant Cell 5 1993, 1039-1048).

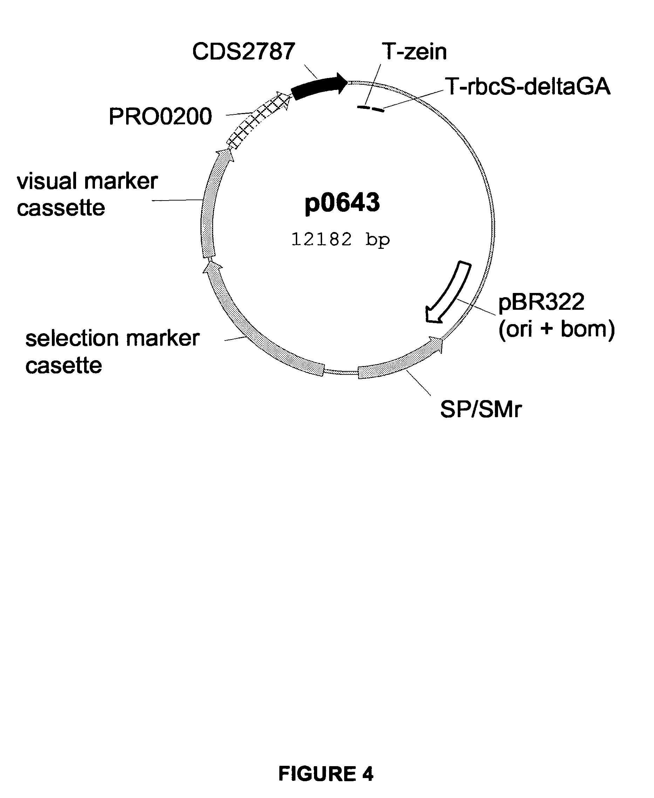

After the LR recombination step, the resulting expression vector p0643 (Figure 4) was transformed into *Agrobacterium* strain LBA4044 and subsequently to *Oryza sativa* plants. Transformed rice plants were allowed to grow and were then examined for the parameters described in Example 3.

***Example 3: Evaluation and Results of OsMADS18 under the control of the rice OSH1 promoter***

Approximately 15 to 20 independent **T0** rice transformants were generated. The primary transformants were transferred from a tissue culture chamber to a greenhouse for growing and harvest of T1 seed. 5 events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. 4 T1 events were further evaluated in the T2 generation following the same evaluation procedure as for the T1 generation but with more individuals per event.

**Statistical analysis: F-test**

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and for an overall effect of the gene, also known as a global gene effect. The threshold for significance for a true global gene effect was set at a 5% probability level for the F-test. A significant F-test value points to a gene effect, meaning that it is not only the presence or position of the gene causing the differences in phenotype.

**Seed-related parameter measurements**

The mature primary panicles were harvested, bagged, barcode-labeled and then dried for three days in an oven at 37°C. The panicles were then threshed and all the seeds were collected and counted. The filled husks were separated from the empty ones using an airblowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) was extrapolated from the number of filled seeds counted and their total weight. Individual seed parameters (including width, length, area, weight) were measured using a custom-made device consisting of two main components, a weighing and imaging device, coupled to software for image analysis.

The tables of results (Tables 5 and 6) below show the p values from the F test for the T1 and T2 generations. The percentage difference between the transgenics and the corresponding nullizygotes is also shown.

The TKW is significantly increased in both the T1 and the T2 generations (Tables 5 and 6). In parallel, an increase in individual seed area is observed, contributing to the observed TKW increase. This increase in seed area is due to a significant increase in individual seed length (Tables 5 and 6), as individual seed width is not significantly changed (data not shown).

**Table 6: Results of the T1 generation**

| | Number of lines showing an increase | % Difference | P value of F test |
|---|---|---|---|
| Thousand kernel weight | 5 out 5 | 4 | 0.0123 |
| Average seed area | 4 out 5 | 3 | 0.0007 |
| Average seed length | 3 out 5 | 2 | 0.0001 |

**Table 7: Results of the T2 generation**

| | Number of lines showing a positive difference | % Difference | P value |
|---|---|---|---|
| Thousand kernel weight | 2 out 4 | 2 | 0.0109 |
| Average seed area | out 4 | 3 | 0.0001 |
| Average seed length | 2 out 4 | 3 | <0.0001 |

## Claims

1. Method for increasing seed yield relative to corresponding wild type plants, comprising introducing through plant transformation and overexpressing in a plant a nucleic acid, encoding an OsMADS18-like polypeptide, which OsMADS18-like polypeptide:

   (i) is a monocot type II MADS-box transcription factor of the SQUAMOSA clade; and
   (ii) has DNA and protein binding activity; and
   (iii) comprises the motif LPPWMLRT (SEQ ID NO: 18) in the last 15 amino acids of the C terminus of the protein, allowing for one amino acid substitution anywhere in the motif but not in the second and fourth positions; and
   (iv) has at least 65% sequence identity to the OsMADS18 protein of SEQ ID NO: 2.

2. Method according to claim 1, wherein said nucleic acid is a portion of an OsMADS18-like nucleic acid of at least 747 nucleotides, which portion encodes a monocot type II MADS-box transcription factor of the SQUAMOSA clade of at least 249 amino acids, having DNA and protein binding activity and comprising:

   (i) the motif LPPWMLRT (SEQ ID NO: 18) in the last 15 amino acids of the C terminus of the protein, allowing for one amino acid substitution anywhere in the motif but not in the second and fourth positions; and/or
   (ii) having at least 65% sequence identity to the OsMADS18 protein of SEQ ID NO: 2.

3. Method according to claim 1, wherein said nucleic acid is a sequence capable of stringently hybridising to an OsMADS18-like nucleic acid, which hybrising sequence or complement thereof encodes a monocot type II MADS-box transcription factor of the SQUAMOSA clade having DNA and protein binding activity and comprising:

   (i) the motif LPPWMLRT (SEQ ID NO: 18) in the last 15 amino acids of the C terminus of the protein, allowing for one amino acid substitution anywhere in the motif but not in the second and fourth positions; and/or
   (ii) having at least 65% sequence identity to the OsMADS18 protein of SEQ ID NO: 2.

4. Method according to any one of claims 1 to 3, wherein said isolated OsMADS18-like nucleic acid is from a monocotyledonous plant.

5. Method according to any one of claims 1 to 4, wherein said nucleic acid encodes an orthologue or paralogue of an OsMADS18 protein of SEQ ID NO: 2, said orthologue or paralogue comprising the following features:

   (i) is a monocot type II MADS-box transcription factor of the SQUAMOSA clade; and
   (ii) has DNA and protein binding activity; and

(iii) comprises the motif LPPWMLRT (SEQ ID NO: 18) in the last 15 amino acids of the protein, allowing for one amino acid substitution anywhere in the motif but not at the second and fourth positions; and
(iv) has at least 65% sequence identity to the OsMADS18 protein of SEQ ID NO: 2.

**6.** Method according to any one of claims 1 to 5, wherein said OsMADS18-like nucleic acid is operably linked to an early shoot apical meristem promoter.

**7.** Method according to claim 6, wherein said early shoot apical meristem promoter is an OSH 1 promoter.

**8.** Method according to any one of claims 1 to 7, wherein said increased seed yield is selected from any one or more of: (i) increased thousand kernel weight (TKW); (ii) increased seed size; (iii) increased seed volume; (iv) increased seed area; (v) increased seed length; and (vi) increased seed biomass.

**9.** Construct comprising:

(a) a nucleic acid encoding an OsMADS18-like polypeptide, which OsMADS18-like polypeptide:

(i) is a monocot type I I MADS-box transcription factor of the SQUAMOSA clade; and
(ii) has DNA and protein binding activity; and
(iii) comprises the motif LPPWMLRT (SEQ ID NO: 18) in the last 15 amino acids of the C terminus of the protein, allowing for one amino acid substitution anywhere in the motif but not in the second and fourth positions; and
(iv) has at least 65% sequence identity to the OsMADS18 protein of SEQ ID NO: 2; and

(b) one or more control sequences capable of driving expression of the nucleic acid sequence of (a), wherein said control sequence is an early shoot apical meristem promoter.

**10.** Construct according to claim 9, wherein said early shoot apical meristem promoter is an OSH1 promoter.

**11.** Construct according to claim 10, wherein said OSH1 promoter is as represented by SEQ ID NO: 22.

**12.** Construct according to any one of claims 9 to 11, for use in a method according to any one of claims 1 to 8.

**13.** Plants comprising a construct according to any one of claims 9 to 12.

**14.** Method for the production of a transgenic plant having increased seed yield relative to corresponding wild type plants, which method comprises:

(a) introducing through plant transformation and expressing in a plant, plant part or plant cell a nucleic acid encoding an OsMADS18-like polypeptide operably linked to an early shoot apical meristem promoter, which OsMADS18-like polypeptide:

(i) is a monocot type I I MADS-box transcription factor of the SQUAMOSA clade; and
(ii) has DNA and protein binding activity; and
(iii) comprises the motif LPPWMLRT (SEQ ID NO: 18) in the last 15 amino acids of the C terminus of the protein, allowing for one amino acid substitution anywhere in the motif but not in the second and fourth positions; and
(iv) has at least 65% sequence identity to the OsMADS18 protein of SEQ ID NO: 2;

(b) cultivating the plant cell under conditions promoting plant growth and development.

**15.** Transgenic plant having increased seed yield relative to corresponding wild type plants, resulting from transformation of a plant with a nucleic acid operably linked to an early shoot apical meristem promoter, which nucleic acid encodes an OsMADS18-like polypeptide that:

(i) is a monocot type II MADS-box transcription factor of the SQUAMOSA clade; and
(ii) has DNA and protein binding activity; and
(iii) comprises the motif LPPWMLRT (SEQ ID NO: 18) in the last 15 amino acids of the C terminus of the protein, allowing for one amino acid substitution anywhere in the motif but not in the second and fourth positions; and
(iv) has at least 65% sequence identity to the OsMADS18 protein of SEQ ID NO: 2.

**16.** Transgenic plant according to claim 13 or 15, wherein said plant is a monocotyledonous plant.

**17.** Harvestable parts of a plant according to any one of claims 13, 15 or 16, comprising an OsMADS18-like nucleic acid encoding an OSMADS18-like polypeptide comprising the following features:

(i) is a monocot type II MADS-box transcription factor of the SQUAMOSA clade; and
(ii) has DNA and protein binding activity; and
(iii) comprises the motif LPPWMLRT (SEQ ID NO: 18) in the last 15 amino acids of the protein, allowing for one amino acid substitution anywhere in the motif but not at the second and fourth positions; and
(iv) has at least 65% sequence identity to the OsMADS18 protein of SEQ ID NO: 2, wherein said OsMADS18-like nucleic is operably linked to an early shoot apical meristem promoter.

**18.** Harvestable parts according to claim 17, wherein said harvestable parts are seeds.

**19.** Use of an OsMADS18-like nucleic acid/gene in increasing seed yield, said nucleic acid/gene encoding an OSMADS18-like polypeptide according to claim 1(i) to (iv), or use of an OsMADS18-like polypeptide according to claim 1(i) to (iv), in increasing seed yield.

**20.** Use according to claim 19, wherein said seed yield includes one or more of the following: increased TKW, increased seed size, increased seed volume, increased seed area, increased seed length and increased seed biomass

**Patentansprüche**

**1.** Verfahren zur Erhöhung des Samenertrags im Vergleich zu entsprechenden Wildtyp-Pflanzen, umfassend das Einführen mittels Pflanzentransformation und das Überexprimieren in eine(r) Pflanze einer Nukleinsäure, die ein OsMADS18-ähnliches Polypeptid codiert, wobei das OsMADS18-ähnliche Polypeptid:

(i) ein Typ II-MADS-Box-Transkriptionsfaktor der SQUAMOSA-Klade von Monokotyledonen ist und
(ii) DNA- und Proteinbindungsaktivität besitzt und
(iii) das Motiv LPPWMLRT (SEQ ID NR: 18) in den letzten 15 Aminosäuren des C-Terminus des Proteins umfasst, wobei eine Aminosäuresubstitution an einer beliebigen Stelle in dem Motiv, jedoch nicht an der zweiten und der vierten Position, zulässig ist, und
(iv) mindestens 65 % Sequenzidentität mit dem OsMADS18-Protein der SEQ ID NR: 2 aufweist.

**2.** Verfahren nach Anspruch 1, wobei die Nukleinsäure ein Teil einer OsMADS18-ähnlichen Nukleinsäure von 747 Nukleotiden ist, wobei der Teil einen Typ II-MADS-Box-Transkriptionsfaktor der SQUAMOSA-Klade von Monokotyledonen von mindestens 249 Aminosäuren codiert, der DNA- und Proteinbindungsaktivität besitzt und Folgendes umfasst:

(i) das Motiv LPPWMLRT (SEQ ID NR: 18) in den letzten 15 Aminosäuren des C-Terminus des Proteins umfasst, wobei eine Aminosäuresubstitution an einer beliebigen Stelle in dem Motiv, jedoch nicht an der zweiten und der vierten Position, zulässig ist, und
(ii) mindestens 65 % Sequenzidentität mit dem OsMADS18-Protein der SEQ ID NR: 2 aufweist.

**3.** Verfahren nach Anspruch 1, wobei die Nukleinsäure eine Sequenz ist, die stringent an eine OsMADS18-ähnliche Nukleinsäure hybridisieren kann, wobei die hybridisierende Sequenz oder ein Komplement davon einen Typ II-MADS-Box-Transkriptionsfaktor der SQUAMOSA-Klade von Monokotyledonen codiert, der DNA- und Proteinbindungsaktivität besitzt und Folgendes umfasst:

(i) das Motiv LPPWMLRT (SEQ ID NR: 18) in den letzten 15 Aminosäuren des C-Terminus des Proteins umfasst, wobei eine Aminosäuresubstitution an einer beliebigen Stelle in dem Motiv, jedoch nicht an der zweiten und der vierten Position, zulässig ist, und
(ii) mindestens 65 % Sequenzidentität mit dem OsMADS18-Protein der SEQ ID NR: 2 aufweist.

4. Verfahren nach einem der Ansprüche 1 bis 3, wobei die isolierte OsMADS18-ähnliche Nukleinsäure aus einer monokotyledonen Pflanze stammt.

5. Verfahren nach einem der Ansprüche 1 bis 4, wobei die Nukleinsäure ein Ortholog oder ein Paralog eines OsMADS18-Proteins der SEQ ID NR: 2 codiert, wobei das Ortholog oder Paralog die folgenden Merkmale umfasst:

(i) e s i st ein Typ II-MADS-Box-Transkriptionsfaktor der SQUAMOSA-Klade von Monokotyledonen und
(ii) besitzt DNA- und Proteinbindungsaktivität und
(iii) umfasst das Motiv LPPWMLRT (SEQ ID NR: 18) in den letzten 15 Aminosäuren des C-Terminus des Proteins, wobei eine Aminosäuresubstitution an einer beliebigen Stelle in dem Motiv, jedoch nicht an der zweiten und der vierten Position, zulässig ist, und
(iv) weist mindestens 65 % Sequenzidentität mit dem OsMADS18-Protein der SEQ ID NR: 2 auf.

6. Verfahren nach einem der Ansprüche 1 bis 5, wobei die OsMADS18-ähnliche Nukleinsäure funktionsfähig mit einem frühen Sprossapikalmeristem-Promotor verknüpft ist.

7. Verfahren nach Anspruch 6, wobei der frühe Sprossapikalmeristem-Promotor ein OSH1-Promotor ist.

8. Verfahren nach einem der Ansprüche 1 bis 7, wobei der erhöhte Samenertrag aus einem oder mehreren der Folgenden ausgewählt ist: (i) erhöhtes Tausendkorngewicht (TKG), (ii) erhöhte Samengröße, (iii) erhöhtes Samenvolumen, (iv) erhöhte Samenfläche, (v) erhöhte Samenlänge und (vi) erhöhte Samenbiomasse.

9. Konstrukt, umfassend:

(a) eine Nukleinsäure, die ein OsMADS18-ähnliches Polypeptid codiert, wobei das OsMADS18-ähnliche Polypeptid:

(i) ein Typ II-MADS-Box-Transkriptionsfaktor der SQUAMOSA-Klade von Monokotyledonen ist und
(ii) DNA- und Proteinbindungsaktivität besitzt und
(iii) das Motiv LPPWMLRT (SEQ ID NR: 18) in den letzten 15 Aminosäuren des C-Terminus des Proteins umfasst, wobei eine Aminosäuresubstitution an einer beliebigen Stelle in dem Motiv, jedoch nicht an der zweiten und der vierten Position, zulässig ist, und
(iv) mindestens 65 % Sequenzidentität mit dem OsMADS18-Protein der SEQ ID NR: 2 aufweist, und

(b) einer oder mehreren Kontrollsequenzen, die die Expression der Nukleinsäuresequenz unter (a) antreiben können, wobei die Kontrollsequenz ein früher Sprossapikalmeristem-Promotor ist.

10. Konstrukt nach Anspruch 9, wobei der frühe Sprossapikalmeristem-Promotor ein OSH1-Promotor ist.

11. Konstrukt nach Anspruch 10, wobei der OSH1-Promotor wie durch die SEQ ID NR: 22 wiedergegeben ist.

12. Konstrukt nach einem der Ansprüche 9 bis 11 für die Verwendung bei einem Verfahren nach einem der Ansprüche 1 bis 8.

13. Pflanzen, die ein Konstrukt nach einem der Ansprüche 9 bis 12 umfassen.

14. Verfahren zur Herstellung einer transgenen Pflanze mit einem erhöhten Samenertrag im Vergleich zu entsprechenden Wildtyp-Pflanzen, wobei das Verfahren Folgendes umfasst:

(a) das Einführen mittels Pflanzentransformation und das Exprimieren in eine (r) Pflanze, eine (n/m) Pflanzenteil oder eine(r) Pflanzenzelle einer Nukleinsäure, die ein OsMADS18-ähnliches Polypeptid codiert, das funktionsfähig mit einem frühen Sprossapikalmeristem-Promotor verknüpft ist, wobei das OsMADS18-ähnliche Polypeptid:

(i) ein Typ II-MADS-Box-Transkriptionsfaktor der SQUAMOSA-Klade von Monokotyledonen ist und
(ii) DNA- und Proteinbindungsaktivität besitzt und

(iii) das Motiv LPPWMLRT (SEQ ID NR: 18) in den letzten 15 Aminosäuren des C-Terminus des Proteins umfasst, wobei eine Aminosäuresubstitution an einer beliebigen Stelle in dem Motiv, jedoch nicht an der zweiten und der vierten Position, zulässig ist, und
(iv) mindestens 65 % Sequenzidentität mit dem OsMADS18-Protein der SEQ ID NR: 2 aufweist,

(b) das Kultivieren der Pflanzenzelle unter Bedingungen, die das Pflanzenwachstum und die Pflanzenentwicklung fördern.

**15.** Transgene Pflanze mit einem erhöhten Samenertrag im Vergleich zu entsprechenden Wildtyp-Pflanzen, die sich durch die Transformation einer Pflanze mit einer Nukleinsäure ergibt, die funktionsfähig mit einem frühen Sprossapikalmeristem-Promotor verknüpft ist, wobei die Nukleinsäure ein OsMADS18-ähnliches Polypeptid codiert, das:

(i) ein Typ II-MADS-Box-Transkriptionsfaktor der SQUAMOSA-Klade von Monokotyledonen ist und
(ii) DNA- und Proteinbindungsaktivität besitzt und
(iii) das Motiv LPPWMLRT (SEQ ID NR: 18) in den letzten 15 Aminosäuren des C-Terminus des Proteins umfasst, wobei eine Aminosäuresubstitution an einer beliebigen Stelle in dem Motiv, jedoch nicht an der zweiten und der vierten Position, zulässig ist, und
(iv) mindestens 65 % Sequenzidentität mit dem OsMADS18-Protein der SEQ ID NR: 2 aufweist.

**16.** Transgene Pflanze nach Anspruch 13 oder 15, wobei die Pflanze eine monokotyledone Pflanze ist.

**17.** Erntbare Teile einer Pflanze nach einem der Ansprüche 13, 15 oder 16, die eine OsMADS18-ähnliche Nukleinsäure umfassen, die ein OsMADS18-ähnliches Polypeptid codiert, das die folgenden Merkmale umfasst:

(i) es ist ein Typ II-MADS-Box-Transkriptionsfaktor der SQUAMOSA-Klade von Monokotyledonen und
(ii) besitzt DNA- und Proteinbindungsaktivität und
(iii) umfasst das Motiv LPPWMLRT (SEQ ID NR: 18) in den letzten 15 Aminosäuren des C-Terminus des Proteins, wobei eine Aminosäuresubstitution an einer beliebigen Stelle in dem Motiv, jedoch nicht an der zweiten und der vierten Position, zulässig ist, und
(iv) weist mindestens 65 % Sequenzidentität mit dem OsMADS18-Protein der SEQ ID NR: 2 auf, wobei die OsMADS18-ähnliche Nukleinsäure funktionsfähig mit einem frühen Sprossapikalmeristem-Promotor verknüpft ist.

**18.** Erntbare Teile nach Anspruch 17, wobei es sich bei den erntbaren Teilen um Samen handelt.

**19.** Verwendung einer OsMADS18-ähnlichen Nukleinsäure/ eines OsMADS18-ähnlichen Gens zur Erhöhung des Samenertrags, wobei die Nukleinsäure/das Gen ein OsMADS18-ähnliches Polypeptid nach Anspruch 1(i) bis (iv) codiert, oder Verwendung eines OsMADS18-ähnlichen Polypeptids nach Anspruch 1(i) bis (iv) zur Erhöhung des Samenertrags.

**20.** Verwendung nach Anspruch 19, wobei der erhöhte Samenertrag eines oder mehrere der Folgenden umfasst: erhöhtes TKG, erhöhte Samengröße, erhöhtes Samenvolumen, erhöhte Samenfläche, erhöhte Samenlänge und erhöhte Samenbiomasse.

## Revendications

**1.** Procédé pour augmenter le rendement en graines par rapport à des plantes correspondantes de type sauvage, comprenant l’introduction, par transformation végétale et surexpression dans une plante, d’un acide nucléique codant pour un polypeptide OsMADS18-like, lequel polypeptide OsMADS18-like

(i) est un facteur de transcription à boîte MADS de type II de monocotylédone du clade de SQUAMOSA ; et
(ii) a une activité de liaison à l’ADN et aux protéines ; et
(iii) comprend le motif LPPWMLRT (SEQ ID NO:18) dans les 15 derniers acides aminés du site C terminal de la protéine, en autorisant une substitution d’acide aminé en un point quelconque du motif mais non pas sur la deuxième et la quatrième positions ; et

(iv) a une identité de séquence d'au moins 65 % avec la protéine OsMADS18 de SEQ ID NO:2.

**2.** Procédé selon la revendication 1, dans lequel l'acide nucléique est une portion d'un acide nucléique OsMADS18-like d'au moins 747 nucléotides, laquelle portion codant pour un facteur de transcription à boîte MADS de type II de monocotylédone du clade de SQUAMOSA d'au moins 249 acides aminés, ayant une activité de liaison à l'ADN et aux protéines, et comprenant :

(i) le motif LPPWMLRT (SEQ ID NO:18) dans les 15 derniers acides aminés du site C terminal de la protéine, permettant une substitution d'acide aminés en un point quelconque du motif mais non sur la deuxième et la quatrième positions ; et/ou
(ii) ayant une identité de séquence d'au moins 65 % avec la protéine OsMADS18 de SEQ ID NO:2.

**3.** Procédé selon la revendication 1, dans lequel l'acide nucléique est une séquence capable de s'hybrider dans des conditions stringentes à un acide nucléique OsMADS18-like, laquelle séquence d'hybridation, ou son complément, codant pour un facteur de transcription à boîte MADS de type II de monocotylédone du clade de SQUAMOSA ayant une activité de liaison à l'ADN et aux protéines, et comprenant :

(i) le motif LPPWMLRT (SEQ ID NO:18) dans les 15 derniers acides aminés du site C terminal de la protéine, permettant une substitution d'acide aminé en un point quelconque du motif mais non sur la deuxième et la quatrième positions ; et/ou
(ii) ayant une identité de séquence d'au moins 65 % avec la protéine OsMADS18 de SEQ ID NO:2.

**4.** Procédé selon l'une quelconque des revendications 1 à 3, dans lequel ledit acide nucléique isolé OsMADS18-like provient d'une plante monocotylédone.

**5.** Procédé selon l'une quelconque des revendications 1 à 4, dans lequel ledit acide nucléique code pour un orthologue ou un paralogue d'une protéine OsMADS18 de SEQ ID NO:2, ledit orthologue ou paralogue comprenant les particularités suivantes :

(i) il s'agit d'un facteur de transcription à boîte MADS de type II de monocotylédone du clade de SQUAMOSA ; et
(ii) il a une activité de liaison à l'ADN et aux protéines ; et
(iii) il comprend le motif LPPWMLRT (SEQ ID NO:18) dans les 15 derniers acides aminés de la protéine, permettant une substitution d'acide aminé en un point quelconque du motif mais non sur la deuxième et la quatrième positions ; et
(iv) il a une identité de séquence d'au moins 65 % avec la protéine OsMADS18 de SEQ ID NO:2.

**6.** Procédé selon l'une quelconque des revendications 1 à 5, dans lequel l'acide nucléique OsMADS18-like est lié de manière opérationnelle à un promoteur du méristème apical d'une pousse anticipée.

**7.** Procédé selon la revendication 6, dans lequel ledit promoteur du méristème apical d'une pousse anticipée est un promoteur OSH1.

**8.** Procédé selon l'une quelconque des revendications 1 à 7, dans lequel ladite augmentation du rendement en graines est choisie parmi un ou plusieurs quelconque

(i) d'une augmentation du poids de mille graines (PMG) ;
(ii) d'une augmentation de la taille des graines ;
(iii) d'une augmentation du volume des graines ;
(iv) d'une augmentation de l'aire des graines ;
(v) d'une augmentation de la longueur des graines ; et
(vi) d'une augmentation de la biomasse des graines.

**9.** Construction comprenant :

(a) un acide nucléique codant pour un polypeptide OsMADS18-like, lequel polypeptide OsMADS18-like

(i) est un facteur de transcription à boîte MADS de type II de monocotylédone du clade de SQUAMOSA ; et
(ii) a une activité de liaison à l'ADN et aux protéines ; et

(iii) comprend le motif LPPWMLRT (SEQ ID NO:18) dans les 15 derniers acides aminés du site C terminal de la protéine, en autorisant une substitution d'acide aminé en un point quelconque du motif mais non pas sur la deuxième et la quatrième positions ; et
(iv) a une identité de séquence d'au moins 65 % avec la protéine OsMADS18 de SEQ ID NO:2 ; et

(b) une ou plusieurs séquences de contrôle, capables de commander l'expression de la séquence d'acide nucléique (a), ladite séquence de contrôle étant un promoteur du méristème apical d'une pousse anticipée.

**10.** Construction selon la revendication 9, dans laquelle ledit promoteur du méristème apical d'une pousse anticipée est un promoteur OSH1.

**11.** Construction selon la revendication 10, dans laquelle le promoteur OSH1 est tel que représenté par SEQ ID NO:22.

**12.** Construction selon l'une quelconque des revendications 9 à 11, pour utilisation dans un procédé selon l'une quelconque des revendications 1 à 8.

**13.** Plantes comprenant une construction selon l'une quelconque des revendications 9 à 12.

**14.** Procédé de production d'une plante transgénique présentant une augmentation du rendement en graines par rapport à des plantes correspondantes de type sauvage, ledit procédé comprenant :

(a) l'introduction, par transformation végétale et expression dans une plante, une partie de plante ou une cellule végétale, d'un acide nucléique codant pour un polypeptide OsMADS18-like lié de manière opérationnelle à un promoteur du méristème apical d'une pousse anticipée, lequel polypeptide OsMADS18-like :

(i) est un facteur de transcription à boîte MADS de type II de monocotylédone du clade de SQUAMOSA ; et
(ii) a une activité de liaison à l'ADN et aux protéines ; et
(iii) comprend le motif LPPWMLRT (SEQ ID NO:18) dans les 15 derniers acides aminés du site C terminal de la protéine, en autorisant une substitution d'acide aminé en un point quelconque du motif mais non pas sur la deuxième et la quatrième positions ; et
(iv) a une identité de séquence d'au moins 65 % avec la protéine OsMADS18 de SEQ ID NO:2 ;

(b) la culture de la cellule végétale dans des conditions favorisant la croissance et le développement de la plante.

**15.** Plante transgénique présentant une augmentation du rendement en graines par rapport à des plantes correspondantes de type sauvage, résultant de la transformation d'une plante avec un acide nucléique lié de manière opérationnelle à un promoteur du méristème apical d'une pousse anticipée, lequel acide nucléique codant pour un polypeptide OsMADS18-like, qui

(i) est un facteur de transcription à boîte MADS de type II de monocotylédone du clade de SQUAMOSA ; et
(ii) a une activité de liaison à l'ADN et aux protéines ; et
(iii) comprend le motif LPPWMLRT (SEQ ID NO:18) dans les 15 derniers acides aminés du site C terminal de la protéine, en autorisant une substitution d'acide aminé en un point quelconque du motif mais non pas sur la deuxième et la quatrième positions ; et
(iv) a une identité de séquence d'au moins 65 % avec la protéine OsMADS18 de SEQ ID NO:2.

**16.** Plante transgénique selon la revendication 13 ou 15, ladite plante étant une plante monocotylédone.

**17.** Parties récoltables d'une plante selon l'une quelconque des revendications 13, 15 ou 16, comprenant un acide nucléique OsMADS18-like codant pour un polypeptide OsMADS18-like, comprenant les particularités suivantes :

(i) il s'agit d'un facteur de transcription à boîte MADS de type II de monocotylédone du clade de SQUAMOSA ; et
(ii) il a une activité de liaison à l'ADN et aux protéines ; et
(iii) il comprend le motif LPPWMLRT (SEQ ID NO:18) dans les 15 derniers acides aminés de la protéine, permettant une substitution d'acide aminé en un point quelconque du motif mais non sur la deuxième et la quatrième positions ; et
(iv) il a une identité de séquence d'au moins 65 % avec la protéine OsMADS18 de SEQ ID NO:2,

où l'acide nucléique OsMADS18-like est lié de manière opérationnelle à un promoteur du méristème apical d'une pousse anticipée.

**18.** Parties récoltables selon la revendication 17, lesdites parties récoltables étant des graines.

**19.** Utilisation d'un acide nucléique/gène OsMADS18-like pour augmenter le rendement en graines, ledit acide nucléique/gène codant pour un polypeptide OsMADS18-like selon la revendication 1(i) à (iv), ou utilisation d'un polypeptide OsMADS18-like selon la revendication 1(i) à (iv), pour augmenter le rendement en graines.

**20.** Utilisation selon la revendication 19, pour laquelle ledit rendement en graines comprend une ou plusieurs d'une augmentation du PMG, d'une augmentation de la taille des graines, d'une augmentation du volume des graines, d'une augmentation de l'aire des graines, d'une augmentation de la longueur des graines et d'une augmentation de la biomasse des graines.

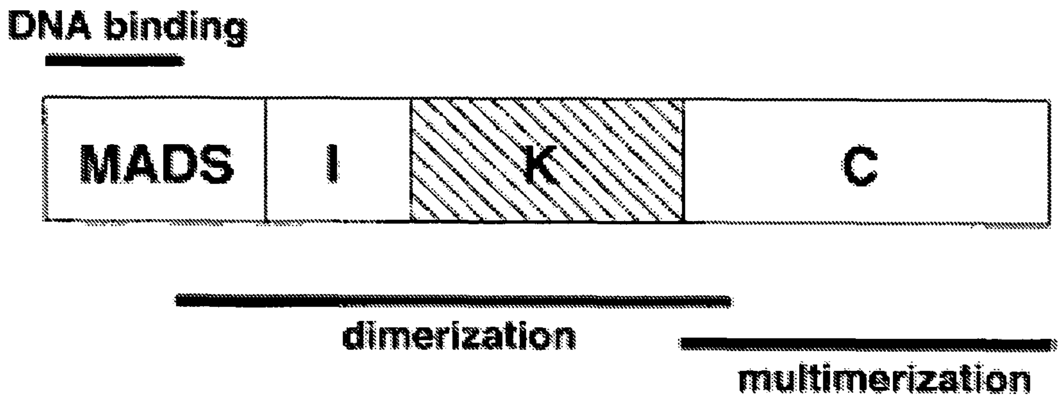


FIGURE 1

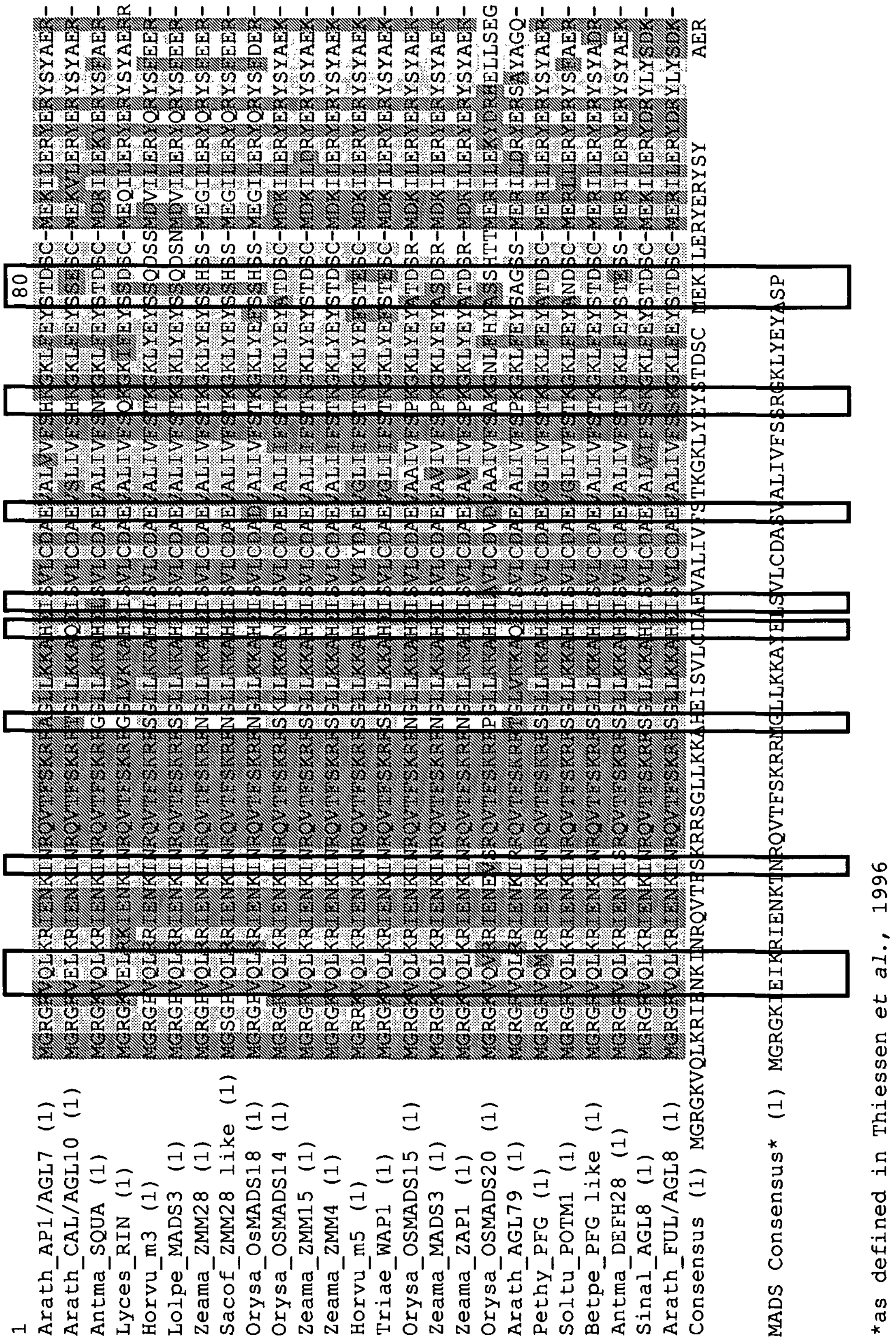
1
80
Arath_AP1/AGL7 (1)
Arath_CAL/AGL10 (1)
Antma_SQUA (1)
Lyces_RIN (1)
Horvu_m3 (1)
Lolpe_MADS3 (1)
Zeama_ZMM28 (1)
Sacof_ZMM28 like (1)
Orysa_OsMADS18 (1)
Orysa_OSMADS14 (1)
Zeama_ZMM15 (1)
Zeama_ZMM4 (1)
Horvu_m5 (1)
Triae_WAP1 (1)
Orysa_OSMADS15 (1)
Zeama_MADS3 (1)
Zeama_ZAP1 (1)
Orysa_OSMADS20 (1)
Arath_AGL79 (1)
Pethy_PFG (1)
Soltu_POTM1 (1)
Betpe_PFG like (1)
Antma_DEFH28 (1)
Sinal_AGL8 (1)
Arath_FUL/AGL8 (1)
Consensus (1)
MADS Consensus* (1)
*as defined in Thiessen et al., 1996

FIGURE 2

```
81                                                                                   160
Arath_AP1/A  GL7 (79) -QLIAPESDVNTNWSMEYNRLKAKIELLERNQRHYLGEDLQAMSPKELQNLEQQLDTALKHIRTRKNQLMYESINELQKK
Arath_CAL/AGL10 (79)  -QLIAPDSHVNTNWSMEYSRLKAKIELLERNQRHYLGEELEPMSLKDLQNLEQQLETALKHIRSRKNQLMNESLNHLQRK
Antma_SQUA (79)       -QLVSNEPQSPANWTLEYSKLKARIELLQRNHRHYMGEDLDSMSLKEIQSLEQQLDTALKNIRTRKNQLLYDSISELQHK
Lyces_RIN (80)        LLANNSESPVQENWSLEYTKLKARIDLLQRNHKHYMGEDLDSMSLKDLQNLEQQLDSALKLIRSRKNQLMHESISELQKK
Horvu_m3 (80)         -AVLDPSTGDQANWGDEYGSLKIKLDALQKSQRQLLGEQLDPLTTKELQQLEQQLDSSLKHIRSRKNQLLFESISELQKK
Lolpe     _MADS3 (80) -AIVDQNIGGQANWGDEFGSLKIKLDALQKSQRQLLGEQLDPLTTKELQQLEQQLDSSLKHIRSRKNQLLFESISELQKK
Zeama_ZMM28 (79)      -AVINPSIEDQANWGDEYVRLKSKLDALQKSQRQLLGEQLSSLTIKELQQLEQQLDSSLKHIRSRKNQLMFDSISALQKK
Sacof_ZMM28 like (79) -AVLDPSIEDQADWGDEYVRLKSKLDALQKSQRHLLGEQLDSLTIKELQQLEQQLDSSLKHIRSRKNQLMFDSISELQKK
Orysa_OsMADS18 (79)   -AVLEPNTEDQENWGDEYGILKSKLDALQKSQRQLLGEQLDTLTTKELQQLEHQLEYSLKHIRSKKNQLLFESISELQKK
Orysa_OSMADS14 (79)   -VLISAESDTQGNWCHEYRKLKAKVETIQKCQKHLMGEDLESLNLKELQQLEQQLENSLKHIRSRKSQLMLESINELQRK
Ze     ama_ZMM15 (79) -VLISAESETQGNWCHEYRKLKAKVETIQKCQKHLMGEDLETLNLKELQQLEQQLESSLKHIRTRKNQLMLESISELQRK
Zeama_ZMM4 (79)       -VLISAEYETQGNWCHEYRKLKAKVETIQKCQKHLMGEDLETLNLKELQQLEQQLESSLKHIRTRKSQLMVESISALQRK
Horvu_m5 (79)         -VLVSSESEIQGNWCHEYRKLKAKVETIQKCQKHLMGEDLESLNLKELQQLEQQLESSLKHIRARKNQLMHESISELQKK
Triae_WAP1 (79)       -VLVSSESEIQGNWCHEYRKLKAKVETIQKCQKHLMGEDLESLNLKELQQLEQQLESSLKHIRSRKNQLMHESISELQKK
Orysa_OSMADS15 (79)   -ALISAESESEGNWCHEYRKLKAKIETIQKCHKHLMGEDHESLNLKELQQLEQQLESSLKHIISRKSHLMLESISELQKK
    Zeama_MADS3 (79)  -ALISAESESEGNWCHEYRKLKAKIETIQRCHKHLMGEDLESLNPKELQQLEQQLESSLKHIRSRKSHLMAESISELQKK
Zeama_ZAP1 (79)       -ALISAESESEGNWCHEYRKLKAKIETIQKCHKHLMGEDLESLNPKELQQLEQQLDSSLKHIRSRKSHLMAESISELQKK
Orysa_OSMADS20 (81)   NNVIEEFPELEGSMSYDHIKLRGRIEALKKSQRNLMGQELDSLTLQDIQQLENQIDTSLNNIRSRKNNLLLKSIAELRQK
Arath_AGL79 (79)      -DIPTPNLDSQGECSTECSKLLRMIDVLQRSLRHLRGEEVDGLSIRDLQGVEMQLDTALKKTRSRKNQLMVESIAQLQKK
Pethy_PFG (79)        -QLVSTDHSSPGSWNLEHAKLKARIEVVQRNQRHYMGEDLDSLSMKDLQNLEQQLDSSLKHIRSRKNQLMHESISELQKK
 Soltu_POTM1 (79)     -QLVPTDHTSPGSWTLEHAKLKARLEVLQRNQKHYVGEDLESLNMKELQNLEHQLDSALKHIRSRKNQLMHESISVLQKQ
Betpe_PFG like (79)   -QLLANDLEQNGSWTLEHAKLKARIEVLQRNQKHFVGEDLDSLSLKELQNLEQQLDSALKHIRSRKNQLMYESISELQRK
Antma_DEFH28 (79)     -KLTSDSHEPEENWCLEYPKLVARIELLERNIRNYVGEDLDHLSMRELQSLEQQLDTALKRTRTRKNQLMHESISQLQKK
Sinal_AGL8 (79)       -QLVGRDISQSENWVLEHAKLKARVEVLEKNKRNFMGEDLDSLSLKELQSLEHQLHAAIKSIRSRKNQAMFESISALQKK
Arath_FUL/AGL8 (79)   -QLVGRDVSQSENWVLEHAKLKARVEVLEKNKRNFMGEDLDSLSLKELQSLEHQLDAAIKSIRSRKNQAMFESISALQKK
Consensus (81)    LIS E E QGNW  EY KLKAKIE LQK QRHLMGEDLDSLSLKELQQLEQQLDSSLKHIRSRKNQLM ESISELQKK
```

**FIGURE 2 (continued)**

```
161                                                                            240
Arath_AP1/AGL  7 (158)   EKAIQEQNSMLSKQIKEREK-------------ILRAQQEQWDQ------------------------QNQGHNMPPPL
Arath_CAL/AGL10 (158)    EKEIQEENSMLTKQIKEREN-------------ILRTKQTQCEQ------------------------LNRSVDDVP--
Antma_SQUA (158)         EKAIQEQNTMLAKKIKEKEKE------------IAQQPQWEHHR------------------------HHTNASIMP--
Lyces_RIN (160)          ERAILEENNMLTKKIKEKDKI-----------VEQQGEWHQQTN---------------------------QVSTST--
Horvu_m3 (159)           EKSLKDQNGVLQKHLVETEKEKNN--------VLSNIHHREQLN------------EATNIHHQEQLSGATTSSPSP--
Lo     lpe_MADS3 (159)   EKSLKDQNGVLQKHLVETEKEKSN--------VLSNIHHREQTNGAANIHRREQMNETTHIHNQEQLNGATTSSPSP--
Zeama_ZMM28 (158)        EKALTDQNGVLQK-FMEAEKEKNK--------ALMNAQLREQQN------------------------GASTSSPSL--
Sacof_ZMM28 like (158)   EKALTDQNGVLQK-LMEAEKEKNN--------ALMNAHLREQQN------------------------GASTSSPSL--
Orysa_OsMADS18 (158)     EKSLKNQNNVLQK-LMETEKEKNN--------AIINTN-REEQN------------------------GATPSTSSP--
Orysa_OSMADS14 (158)     EKSLQEENKVLQKELVEKQK------------VQKQQVQWDQTQP-----------------------QTSSSSSSFMM
Zeama_ZMM15 (158)        EKSLQEENKVLQKELAEKQK------------AQRKQVQWGQTQQ-----------------------QTSSSSSCFVI
Zeama_ZMM4 (158)         EKSLQEENKVLQKELAEKQK------------DQRQQVQRDQTQQ-----------------------QTSSSSTSFML
Horvu_m5 (158)           ERSLQEENKVLQKELVEKQK---------------AQAAQQDQTQP----------------------QTSSSSSSFMM
Triae_WAP1 (158)         ERSLQEENKVLQKELVEKQK---------------AQAAQQDQTQP----------------------QTSSSSSSFMM
Orysa_OSMADS15 (158)     ERSLQEENKALQKELVERQKNVRGQQQVGQWDQTQVQAQAQAQPQA----------------------QTSSSSSS-ML
Zeama_MADS3 (158)        ERSLQEENKILQKELSERQKAVASRQ---QQQQQ-VQWDQQTQVQV----------------------QTSSSSSSFMM
Zeama_ZAP1 (158)         ERSLQEENKALQKELAERQKAVASRQ---QQQQQQVQWDQQTHAQA----------------------QTSSSSSSFMM
Orysa_OSMADS20 (161)     EKLLMEKNTILEKKITELET--------------------------------------------------LHTCIRASPTK
Arath_AGL79 (158)        EKELKELKKQLTKKAGEKE-------------DFQTQNLSHD--------------------------LASLATPPFES
Pethy_PFG (158)         DKSLQEQNNLLSKKVKEREKE------------LAQQTQWEQQNN----------------------HHEINSSSSFVL
Soltu_POTM1 (158)        DRALQEQNNQLSKKVKEREKE------------VAQQNQWDQQN-----------------------HEIN-SSTFVL
Betpe_PFG like (158)     DKALQEQNNVLAKKVKEKEKE------------LAQQAQWEQQS------------------------HTLDSVPSLL
Antma_DEFH28 (158)       ERSLQDQNNILAKKIKDNEKQ------------QNEKQQDVHEGF-----------------------AQSSSSINMLL
Si      nal_AGL8 (158)   DKVLQDHNNALLKKIKEREK--------------NTVHQEVQLIQ-----------------------CSNNSS--LL
Arath_FUL/AGL8 (158)     DKALQDHNNSLLKKIKEREK--------------KTGQQEGQLVQ-----------------------CSNSSS----
Consensus (161) EKSLQE N VLQK L EREK                Q  Q  Q Q                          SSSSSS ML
```

**FIGURE 2 (continued)**

Dicot AP1 clade | Monocot (LPPWMLRT, LPPWMLSH) | Dicot FUL clade

```
241                                                   308
Arath_AP1/AGL7 (200)     PPQQHQIQHPYMLSHQPSPFLNMGGLYQEDDPMAM-----------RRNDLELTLEPVYNCNLGCFAA
Arath_CAL/AGL10 (198)    QPQPFQHPHLYMIAHQTSPFLNMGGLYQGEDQTAM-----------RRNNLDLTLEPIYNY-LGCYAA
Antma_SQUA (199)         -------PPPQYSMAPQFPCINVGNTYEGEGANED-----------RRNELDLTLDSLYSCHLGCFAA
Lyces_RIN (199)          -----------SFLLQPHQCLNMGGNYQDEVAEAR-----------RNNELDLNLDSLYPLYNMNKHL
Horvu_m3 (216)           --TPPTAQDSMAPPNIGPYQSR--GGGDPEPQPSPAQAN------------NSNLPPWMLRTIGNR
Lolpe_MADS3 (228)        --TPVAVLDSVATLNIGSSQSREAAGEEPESQPSPAQAN------------SGKLPPWMLRTISNR
Zeama_ZMM28 (202)        --SPPIVPDSMPTLNIGPCQHRGAAESESEPSPAPAQAN------------RGNLPPWMLRTVK
Sacof_ZMM28 like (202)   --SPPMVPDSMPTLNIGSCQPRGPGESEPEPSPAPVQAN------------SGNLPPWMLRTVSNR
Orysa_OsMADS18 (201)     --TPVTAPDPIPTTNNSQSQPRGSGESEA--QPSPAQAG------------NSKLPPWMLRTSHT
Orysa_OSMADS14 (202)     -------REALPTTNISNYPAAAGERIEDVAAGQPQ-------------HERIGLPPWMLSHINGE
Zeama_ZMM15 (202)        -------REAAPTTNISIFPVAAGGRLVEGAAAQP--------------QARVGLPPWMLSHLSS
Zeama_ZMM4 (202)         -------REAAPTTNVSIFPVAAGGRVVEGAAAQP--------------QARVGLPPWMLSHLSC
Horvu_       m5 (200)    -------RDAPPVADTSNHPAAAGERAEDVAVQPQV-------------PLRTALPLWMVSHLNG
Triae_WAP1 (200)         -------RDAPPAAATSIHPAAAGERAGDAAVQPQA-------------PPRTGLPLWMVSHLNG
Orysa_OSMADS15 (214)     ---R-DQQALLPPQNICYPPVMMGERNDAAAAAAVAAQG-------QVQLRIGGLPPWMLSHLNA
   Zeama_MADS3 (211)     ---RQDQQGLPPPQNICFPPLSIGERGE--EVAAAAQQQLPPPGQAQPQLRIAGLPPWMLSHLNA
Zeama_ZAP1 (212)         ---RQDQQGLPPPHNICFPPLTMGDRGEELAAAAAAQQQQPLPGQAQPQLRIAGLPPWMLSHLNA
Orysa_OSMADS20 (192)     AAAPPACNTADAFVPNLNICCGDSGEPETVTAPLGWTSS------------NNGLPWWMLQSSSNGKS
Arath_AGL79 (198)        PHELRRTISPPPPPLSSGDTSQRDGVGEVAAGTLIRRTN-------------ATLPHWMPQLTGE
Pethy_PFG (203)          PQPLDSPHLGEAYQST-----VDNGEVEGAS--QQQP--------------ANTMPPWMLRHLNG
Soltu_POTM1 (200)        PQQLDSPHLGEAYQNTN---VVDNGEVEGGNSSQQQGAA-----------NNTVMPQWMLRHLNG
Betpe_PFG like (200)     PQPLQSS-------LNIGGSQQARGNGRVDEGTPPHRA-------------NALLPPWMLRHLNQ
Antma_DEFH28 (202)       QPPATQLHAVPCLPISGGFQQTVRVEEGGDRTRIADSR--------------SHIPPWILQHVNQ
Sinal_AGL8 (197)         QPQYCLTS------SRDGFVGRVEGENE-GASSLAEPN--------------SLLPAWMLRPTTNE
Arath_FUL/AGL8 (195)     ----VLLPQYCVTSSRDGFVERVGGENG-GASSLTEPN--------------SLLPAWMLRPTTTNE
Consensus (241)                          GG  E   A                    LPPWML HL
```

**FIGURE 2 (continued)**

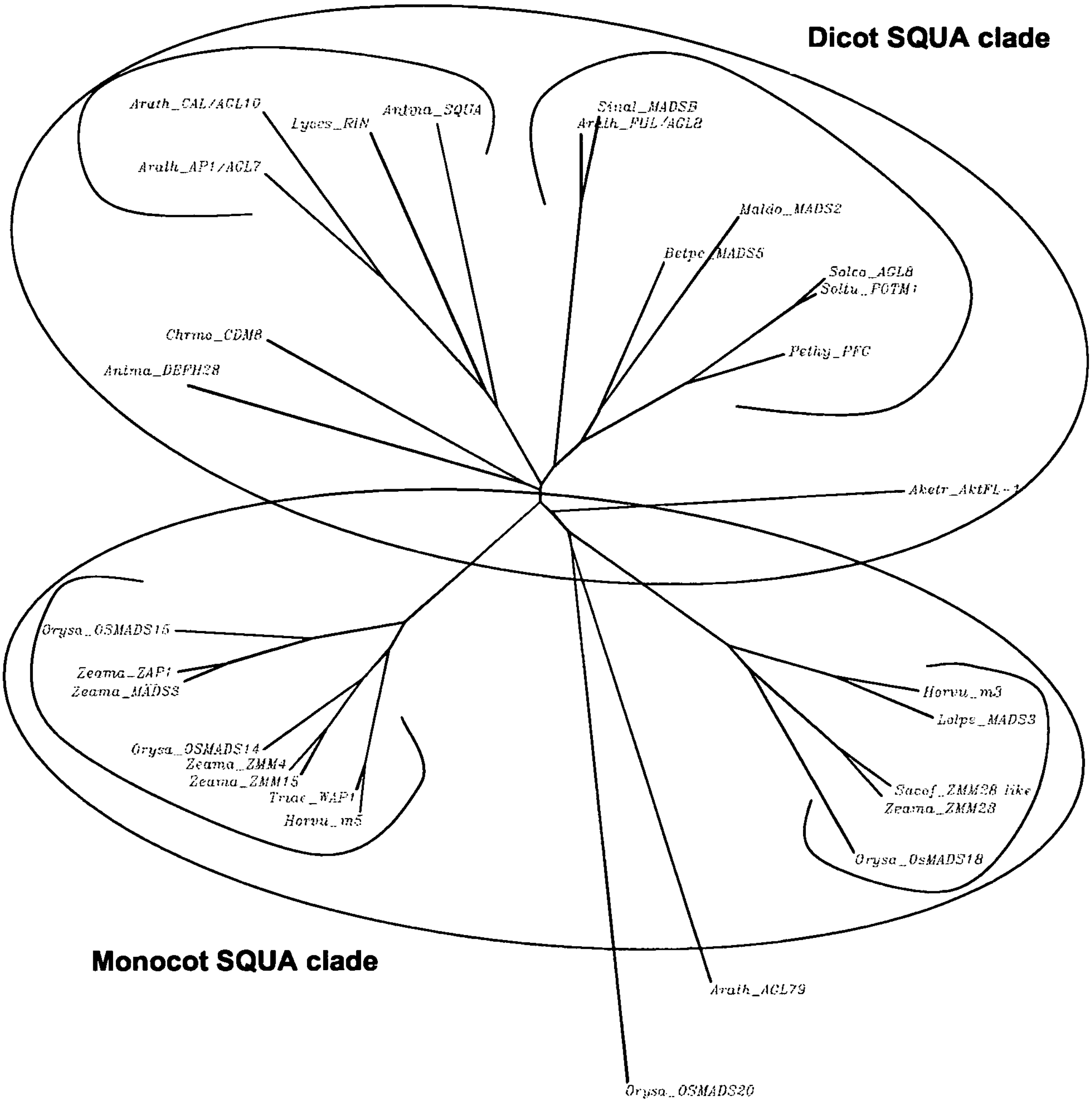
Dicot SQUA clade
Arath_CAL/AGL10
Lyces_RIN
Antma_SQUA
Sinal_MADSB
Arath_FUL/AGL8
Arath_AP1/AGL7
Maldo_MADS2
Betpe_MADS5
Solco_AGL8
Soltu_POTM1
Chrmo_CDM8
Antma_DEFH28
Pethy_PFG
Aketr_AktFL-1
Orysa_OSMADS15
Zeama_ZAP1
Zeama_MADS3
Orysa_OSMADS14
Zeama_ZMM4
Zeama_ZMM15
Triae_WAP1
Horvu_m5
Horvu_m3
Lolpe_MADS3
Sacof_ZMM28 like
Zeama_ZMM28
Orysa_OsMADS18
Monocot SQUA clade
Arath_AGL79
Orysa_OSMADS20

**FIGURE 3**

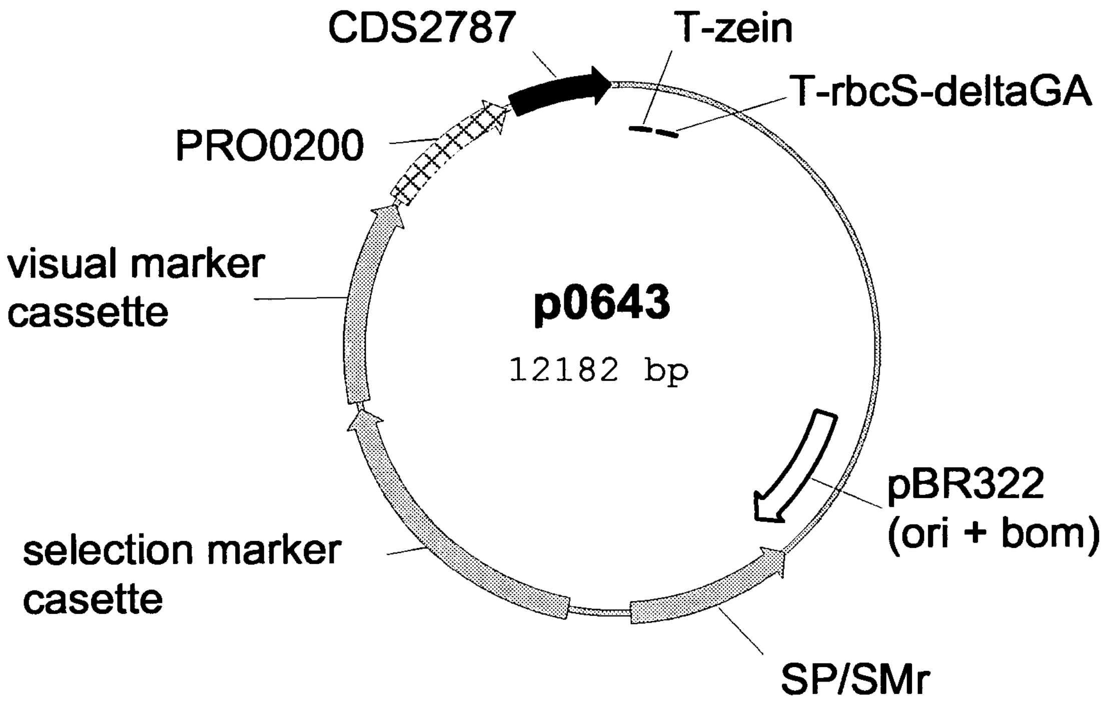
CDS2787
T-zein
T-rbcS-deltaGA
PRO0200
visual marker cassette
p0643
12182 bp
pBR322 (ori + bom)
selection marker casette
SP/SMr

FIGURE 4

**seq id no 01: *Oryza sativa* OsMADS18 DNA (AF091458)**

ATGGGGAGAGGGCCGGTGCAGCTGCGGCGGATCGAGAACAAGATAAACAGGCAGGTGACCTT
CTCCAAGCGGAGGAACGGGCTGCTGAAGAAGGCGCACGAGATCTCCGTGCTCTGTGACGCCG
ACGTCGCGCTCATCGTCTTCTCCACCAAGGGCAAGCTCTACGAGTTCTCCAGCCACTCCAGT
ATGGAAGGGATCCTTGAACGCTACCAGCGTTACTCGTTTGATGAAAGAGCCGTACTGGAGCC
AAATACTGAGGACCAGGAAAACTGGGGTGATGAATATGGAATTTTGAAGTCCAAACTGGATG
CACTTCAGAAGAGCCAAAGGCAACTCTTAGGTGAACAATTGGACACACTAACAATAAAAGAA
CTCCAGCAATTGGAACATCAACTGGAATATTCTCTGAAGCATATAAGATCAAAAAAGAATCA
GCTTCTGTTTGAATCAATTTCTGAGCTTCAGAAGAAGGAAAAGTCACTTAAAAACCAGAATA
ATGTTCTGCAAAAGCTCATGGAGACAGAAAAGGAGAAAAACAATGCTATAATAAACACTAAC
CGGGAGGAGCAAAATGGAGCAACACCAAGCACATCATCACCAACACCAGTGACGGCTCCAGA
TCCCATCCCGACAACAAATAACAGTCAAAGCCAACCAAGAGGATCAGGGGAGTCAGAAGCTC
AACCGTCTCCGGCACAAGCAGGCAACAGCAAGCTTCCGCCATGGATGCTCCGGACAAGTCAC
ACATGA

**seq id no 02: *Oryza sativa* OsMADS18 protein**

MGRGPVQLRRIENKINRQVTFSKRRNGLLKKAHEISVLCDADVALIVFSTKGKLYEFSSHSS
MEGILERYQRYSFDERAVLEPNTEDQENWGDEYGILKSKLDALQKSQRQLLGEQLDTLTIKE
LQQLEHQLEYSLKHIRSKKNQLLFESISELQKKEKSLKNQNNVLQKLMETEKEKNNAIINTN
REEQNGATPSTSSPTPVTAPDPIPTTNNSQSQPRGSGESEAQPSPAQAGNSKLPPWMLRTSH
T

**seq id no 03: *Zea mays* ZMM28 (or m28) DNA (AJ430695)**

ATGGGGCGGGGGCCGGTGCAGCTGCGCCGGATCGAGAACAAGATCAACCGCCAGGTGACCTT
CTCCAAGCGCCGGAACGGGCTGCTGAAGAAGGCCCACGAGATCTCCGTGCTCTGCGACGCAG
AGGTCGCGCTCATCGTCTTCTCCACTAAGGGGAAGCTCTACGAGTACTCTAGCCATTCCAGC
ATGGAAGGCATTCTTGAGCGTTACCAGCGTTACTCATTTGAAGAAAGGGCAGTACTTAACCC
AAGTATTGAAGACCAGGCAAATTGGGGAGATGAATATGTCCGGTTAAAATCCAAACTTGATG
CACTTCAGAAGAGTCAAAGGCAGCTGTTAGGAGAACAATTGAGTTCACTGACCATAAAAGAA
CTCCAGCAACTGGAGCAACAACTGGACAGTTCTTTGAAGCATATTAGGTCAAGAAAGAATCA
GCTCATGTTCGATTCAATTTCCGCGCTTCAGAAAAAGGAGAAAGCACTTACAGATCAAAACG
GTGTCCTGCAAAAGTTCATGGAGGCAGAGAAGGAGAAAAACAAGGCTTTGATGAACGCGCAG
CTCCGGGAGCAGCAAAATGGAGCATCAACAAGCTCCCCATCACTTTCACCACCAATAGTTCC
AGATTCCATGCCAACTCTAAATATAGGGCCATGTCAACATAGAGGGGCAGCAGAATCTGAGT
CTGAACCGTCTCCTGCTCCTGCACAAGCAAACAGGGGCAACCTGCCACCATGGATGCTCCGC
ACTGTCAAGTAA

## FIGURE 5

**seq id no 04:** ***Zea mays*** **ZMM28 protein**

MGRGPVQLRRIENKINRQVTFSKRRNGLLKKAHEISVLCDAEVALIVFSTKGKLYEYSSHSS
MEGILERYQRYSFEERAVLNPSIEDQANWGDEYVRLKSKLDALQKSQRQLLGEQLSSLTIKE
LQQLEQQLDSSLKHIRSRKNQLMFDSISALQKKEKALTDQNGVLQKFMEAEKEKNKALMNAQ
LREQQNGASTSSPSLSPPIVPDSMPTLNIGPCQHRGAAESESEPSPAPAQANRGNLPPWMLR
TVK

**seq id no 05:** ***Lolium perenne*** **MADS3 DNA (AY198328)**

ATGGGGCGCGGGCCGGTGCAGCTGCGGCGGATCGAGAACAAGATAAACCGCCAGGTCACCTT
CTCCAAGCGCCGGAGCGGGCTGCTCAAGAAGGCGCACGAGATCTCCGTGCTCTGCGACGCTG
AGGTCGCGCTCATCGTATTCTCCACCAAGGGAAAGCTCTACGAGTACTCCAGCCAGGACAGT
AACATGGATGTCATTCTTGAACGTTACCAGCGTTACTCATTCGAAGAAAGAGCTATAGTGGA
TCAAAATATTGGAGGCCAGGCAAATTGGGGAGATGAATTTGGAAGTTTGAAAATTAAACTCG
ATGCACTCCAGAAGAGTCAAAGGCAACTCTTAGGTGAACAATTGGACCCACTGACCACAAAA
GAACTTCAACAATTGGAACAGCAGCTAGATAGTTCTTTGAAGCACATAAGGTCAAGAAAGAA
TCAGCTTCTGTTTGAGTCAATATCTGAGCTTCAGAAGAAGGAGAAGTCACTTAAAGATCAGA
ATGGCGTCCTGCAGAAGCACCTCGTGGAGACAGAAAAGGAGAAAAGTAACGTTTTATCGAAT
ATTCATCACCGAGAGCAGACGAATGGAGCAGCAAATATTCATCGCCGGGAGCAGATGAATGA
AACAACACATATTCATAACCAGGAGCAGCTCAATGGAGCAACAACAAGCTCACCGTCACCTA
CACCAGTGGCGGTCCTAGATTCCGTGGCAACTCTAAATATTGGGTCATCTCAATCTAGAGAA
GCAGCAGGAGAGGAGCCAGAATCTCAGCCGTCCCCGGCACAAGCAAACAGCGGCAAGCTACC
ACCATGGATGCTCCGCACCATCAGTAACAGATGA

**seq id no 06:** ***Lolium perenne*** **MADS3 protein**

MGRGPVQLRRIENKINRQVTFSKRRSGLLKKAHEISVLCDAEVALIVFSTKGKLYEYSSQDS
NMDVILERYQRYSFEERAIVDQNIGGQANWGDEFGSLKIKLDALQKSQRQLLGEQLDPLTTK
ELQQLEQQLDSSLKHIRSRKNQLLFESISELQKKEKSLKDQNGVLQKHLVETEKEKSNVLSN
IHHREQTNGAANIHRREQMNETTHIHNQEQLNGATTSSPSPTPVAVLDSVATLNIGSSQSRE
AAGEEPESQPSPAQANSGKLPPWMLRTISNR

**FIGURE 5 (continued)**

**seq id no 07: *Hordeum vulgare* m3 DNA (AJ249143)**

ATGGGGCGCGGGCCGGTGCAGCTGCGGCGGATCGAGAACAAGATAAACCGGCAGGTGACCTT
CTCCAAGCGGCGCAGCGGGCTGCTTAAGAAGGCGCACGAGATCTCCGTGCTCTGCGACGCCG
AGGTCGCGCTCATCGTCTTCTCCACCAAGGGCAAGCTATACGAGTACTCCAGCCAGGACAGT
AGCATGGATGTGATTCTTGAACGTTACCAACGTTACTCATTTGAAGAAAGAGCTGTACTGGA
TCCAAGTACTGGAGACCAGGCAAATTGGGGAGACGAATATGGAAGTTTGAAGATAAAACTCG
ATGCACTCCAGAAGAGTCAAAGGCAACTCTTAGGTGAACAATTGGACCCACTCACCACAAAA
GAACTTCAACAGTTGGAACAACAGCTTGATAGTTCTTTGAAGCACATAAGGTCAAGAAAGAA
TCAACTTCTCTTTGAGTCAATATCTGAGCTTCAAAAGAAGGAGAAATCATTAAAAGATCAGA
ATGGCGTCCTGCAGAAGCACCTCGTAGAGACAGAAAAGGAGAAAAATAACGTTTTGTCAAAT
ATTCATCACCGGGAGCAGTTGAATGAAGCAACAAATATTCATCACCAGGAGCAGCTGAGTGG
AGCAACAACAAGCTCACCGTCACCTACACCACCGACGGCCCAAGATTCCATGGCACCTCCAA
ATATTGGGCCATATCAATCTAGAGGAGGAGGGGATCCAGAACCTCAGCCGTCACCAGCACAA
GCAAACAACAGCAATCTGCCACCGTGGATGCTCCGCACCATCGGCAACAGATGA

**seq id no 08: *Hordeum vulgare* m3 protein**

MGRGPVQLRRIENKINRQVTFSKRRSGLLKKAHEISVLCDAEVALIVFSTKGKLYEYSSQDS
SMDVILERYQRYSFEERAVLDPSTGDQANWGDEYGSLKIKLDALQKSQRQLLGEQLDPLTTK
ELQQLEQQLDSSLKHIRSRKNQLLFESISELQKKEKSLKDQNGVLQKHLVETEKEKNNVLSN
IHHREQLNEATNIHHQEQLSGATTSSPSPTPPTAQDSMAPPNIGPYQSRGGGDPEPQPSPAQ
ANNSNLPPWMLRTIGN

**seq id no 09: Saccharum officinarum ZMM28-like partial DNA (EST CA285442)**

CGCACCACTCGAGTCGAGAGACGAGAGCCACCTGATCCATCCTTCCCCGTCTTCCTCTTCCC
ACACGTCCCCATCGCCATTGCTGCGCCGAGAGTGAGCGGGAGAGGGTAGGTGTCGAGGCGGC
GGAGATGGGGAGCGGGCCGGTGCAGCTGCGCCGGATCGAGAACAAGATCAACCGCCAGGTGA
CCTTCTCCAAGCGCCGGAACGGGCTGCTGAAGAAGGCCCACGAGATCTCCGTGCTCTGCGAT
GCAGAGGTCGCGCTCATCGTCTTCTCCACTAAGGGGAAGCTCTACGAGTACTCTAGCCATTC
CAGCATGGAAGGCATTCTTGAGCGTTACCAGCGGTACTCATTTGAAGAAAGAGCAGTACTTG
ACCCAAGTATTGAAGACCAGGCAGATTGGGGAGATGAATATGTCCGGTTAAAATCCAAACTT
GATGCACTTCAGAAGAGTCAAAGGCATCTGCTAGGAGAACAATTGGATTCACTGACCATAAA
AGAACTCCAGCAACTGGAGCAACAATTGGACAGTTCTTTGAAGCATATTAGGTCAAGAAAGA
ATCAGCTCATGTTCGATTCAATTTCTGAGCTTCAGAAAAAGGAGAAAGCACTTACAGATCAN
AATGGTGTCCTGCAAAAGCTCATGGAGGCAGAGAAAGAGAANAACAATGCTTTAATGAACGC
ACACCTNCGGGAGCAGCAAAATGGAGCATCAACAGGCTCCCCATA

**FIGURE 5 (continued)**

**seq id no 10: Saccharum officinarum ZMM28-like partial DNA (EST CA200888)**

TTCTTGAGCGTTACCAGCGGTACTCATTTGAAGAAAGAGCAGTACTTGACCCAAGTATTGAA
GACCAGGCAGATTGGGGAGATGAATATGTCCAGTTAAAATCCAAACTTGATGCACTTCAGAA
GAGTCAAAGACATCTGTTAGGAGAACAATTGGATTCACTGACCATAAAAGAACTCCAGCAAC
TGGAGCAACAATTGGACAGTTCTTTGAAGCATATTAGGTCAAGAAAGAATCAGCTCATGTTC
GATTCAATTTCCGAGCTTCAGAAAAAGGAGAAAGCACTTACAGATCAAAATGGTGTCCTGCA
AAAGCTCATGGAGGCAGAGAAGGAGAAAAACAATGCTTTAATGAACGCACACCTCCGGGAGC
AGCAAAATGTAGCATCAACAAGCTCCCCATCACTGTCACCACCAATGGTTCCAGATTCCATG
CCAACTCTAAATATTGGGTCATGTCAACCTAGAGGGCCAGGAGAATCTGAGCCTGAACCGTC
TCCTGCTCCTGTACAAGCAAACAGCGGCAACCTGCCACCATGGATGCTCCGCACTGTCAGTA
ACAGATGAGCTCTTCCCAATGCAGTTTTGCGGCTGAACACGACAGCAACACACCTGCCACCG
ACACGCCACGGTGACCCAGGCATCAACCGATGCATGATCCCTCTCGATCCTGTGATCCAA

**seq id no 11: Saccharum officinarum ZMM28-like partial DNA (EST CA247266)**

CTTATTTTGCAGCATGGAAGGCATTCTTGAGCGTTACCAGCGGTACTCATTTGAAGAAAGAG
CAGTACTTGACCCAAGTATTGAAGACCAGGCAGATTGGGGAGATGAATATGTCCAGTTAAAA
TCCAAACTTGATGCACTTCAGAAGAGTCAAAGACATCTGTTAGGAGAACAATTGGATTCACT
GACCATAAAAGAACTCCAGCAACTGGAGCAACAATTGGACAGTTCTTTGAAGCATATTAGGT
CAAGAAAGAATCAGCTCATGTTCGATTCAATTTCCGAGCTTCAGAAAAAGGAGAAAGCACTT
ACAGATCAAAATGGTGTCCTGCAAAAGCTCATGGAGGCAGAGAAGGAGAAAAACAATGCTTT
AATGAACGCACACCTCCGGGAGCAGCAAAATGTAGCATCAACAAGCTCCCCATCACTGTCAC
CACCAATGGTTCCAGATTCCATGCCAACTCTAAATATTGGGATGTCAAC

**seq id no 12: Saccharum officinarum ZMM28-like partial DNA (EST CA282968)**

CAGTACTTGACCCAAGTATTAAAGACCAGGCAGATTGGGGAGATGAATATGTCCGGTTAAAA
TCCAAACTTGATGCACTTCAGAAGAGTCAAAGGCATCTGTTAGGAGAACAATTGGATTCACT
GACCATAAAAGAACTCCAGCAACTGGAGCAACAATTGGACAGTTCTTTGAAGCATATTAGGT
CAAGAAAGAATCAGCTCATGTTCGATTCAATTTCCGAGCTTCAGAAAAAGGAGAAAGCACTT
ACAGATCAAAATGGTGTCCTGCAAAAGCTCATGGAGGCAGAGAAGGAGAAAAACAATGCTTT
AATGAACGCACACCTCCGGGAGCAGCAAAATGTAGCATCAACAAGCTCCCCATCACTGTCAC
CACCAATGGTTCCAGATTCCATGCCAACTCTAAATATTGGGTCATGTCAACCTAGAGGGCCA
GGAGAATCTGAGCCTGAACCGTCTCCTGCTCCTGTACAAGCAAACAGCGGCAACCTGCCACC
ATGGATGCTCCGCACTGTCAGTAACAGATGAGCTCTTCCCAATGCAGTTTTGCGGCTGAACA
CGACAGCAACACACCTGCCACCGACACGCCACGGTGAACCAGGCATCAACCGATGCATGATC
CCTCTCGATCCTGTGATCCAATACCGGGCCGTAGTACTACTAATTAAAGTCCAGGCCCCGCT
CAAATTGCTTTACATGGTTGATTGAAAGTTCT

**FIGURE 5 (continued)**

**seq id no 13: Saccharum officinarum ZMM28-like partial DNA (EST CA299090)**

GAAGCTCTACGAGTACTCTAGCCATTCCAGCATGGAAGGCATTCTTGAGCGTTACCAGCGGT
GCTCATTTGAAGAAAGAGCAGTACTTGACCCAAGTATTGAAGACCAGGCAGATTGGGGAGAT
GAATATGTCCGGTTAAAATCCAAACTTGATGCACTTCAGAAGAGTCAAAGGCATCTGTTAGG
AGAACAATTGGATTCACTGACCATAAAAGAACTCCAGCAACTGGAGCAACAATTGGACAGTT
CTTTGAAGCATATTAGGTCAAGAAAGAATCAGCTCATGTTCGATTCAATTTCCGAGCTTCAG
AAAAAGGAGAAAGCACTTACAGATCAAAATGGTGTCCTGCAAAAGCTCATGGAGGCAGAGAA
GGAGAAAAACAATGCTTTAATGAACGCACACCTCCGGGAGCAGCAAAATGGAGCATCAACAA
GCTCCCCATCACTGTCACCACCAATGGTTCCAGATTCCATGCCAACTCTAAATATTGGGTCA
TGTCAACCTAGAGGGCCAGGAGAATCTGAGCCTGAACCGTCTCCTGCTCCTGTACAAGCAAA
CAGCGGGAACCTGCCACCATGGATACTCCGCACTGTCAGTAACAGATGAGCTCTTTCCAATG
CAGTTNTGCGGCTGAACTCGACAGCAACAC

**seq id no 14: Saccharum officinarum ZMM28-like reconstituted DNA, version 1 (change with seq id no 16 in bold)**

ATGGGGAGCGGGCCGGTGCAGCTGCGCCGGATCGAGAACAAGATCAACCGCCAGGTGACCTT
CTCCAAGCGCCGGAACGGGCTGCTGAAGAAGGCCCACGAGATCTCCGTGCTCTGCGATGCAG
AGGTCGCGCTCATCGTCTTCTCCACTAAGGGGAAGCTCTACGAGTACTCTAGCCATTCCAGC
ATGGAAGGCATTCTTGAGCGTTACCAGCGGTACTCATTTGAAGAAAGAGCAGTACTTGACCC
AAGTATTGAAGACCAGGCAGATTGGGGAGATGAATATGTCCGGTTAAAATCCAAACTTGATG
CACTTCAGAAGAGTCAAAGGCATCTGTTAGGAGAACAATTGGATTCACTGACCATAAAAGAA
CTCCAGCAACTGGAGCAACAATTGGACAGTTCTTTGAAGCATATTAGGTCAAGAAAGAATCA
GCTCATGTTCGATTCAATTTCCGAGCTTCAGAAAAAGGAGAAAGCACTTACAGATCAAAATG
GTGTCCTGCAAAAGCTCATGGAGGCAGAGAAGGAGAAAAACAATGCTTTAATGAACGCACAC
CTCCGGGAGCAGCAAAATG**G**AGCATCAACAAGCTCCCCATCACTGTCACCACCAATGGTTCC
AGATTCCATGCCAACTCTAAATATTGGGTCATGTCAACCTAGAGGGCCAGGAGAATCTGAGC
CTGAACCGTCTCCTGCTCCTGTACAAGCAAACAGCGGCAACCTGCCACCATGGATGCTCCGC
ACTGTCAGTAACAGATGA

**seq id no 15: Saccharum officinarum ZMM28-like reconstituted protein, version 1 (change with seq id no 17 in bold)**

MGSGPVQLRRIENKINRQVTFSKRRNGLLKKAHEISVLCDAEVALIVFSTKGKLYEYSSHSS
MEGILERYQRYSFEERAVLDPSIEDQADWGDEYVRLKSKLDALQKSQRHLLGEQLDSLTIKE
LQQLEQQLDSSLKHIRSRKNQLMFDSISELQKKEKALTDQNGVLQKLMEAEKEKNNALMNAH
LREQQN**G**ASTSSPSLSPPMVPDSMPTLNIGSCQPRGPGESEPEPSPAPVQANSGNLPPWMLR
TVSNR

## FIGURE 5 (continued)

**seq id no 16: Saccharum officinarum ZMM28-like reconstituted DNA, version 2 (change with seq id no 14 in bold)**

ATGGGGAGCGGGCCGGTGCAGCTGCGCCGGATCGAGAACAAGATCAACCGCCAGGTGACCTT
CTCCAAGCGCCGGAACGGGCTGCTGAAGAAGGCCCACGAGATCTCCGTGCTCTGCGATGCAG
AGGTCGCGCTCATCGTCTTCTCCACTAAGGGGAAGCTCTACGAGTACTCTAGCCATTCCAGC
ATGGAAGGCATTCTTGAGCGTTACCAGCGGTACTCATTTGAAGAAAGAGCAGTACTTGACCC
AAGTATTGAAGACCAGGCAGATTGGGGAGATGAATATGTCCGGTTAAAATCCAAACTTGATG
CACTTCAGAAGAGTCAAAGGCATCTGTTAGGAGAACAATTGGATTCACTGACCATAAAAGAA
CTCCAGCAACTGGAGCAACAATTGGACAGTTCTTTGAAGCATATTAGGTCAAGAAAGAATCA
GCTCATGTTCGATTCAATTTCCGAGCTTCAGAAAAAGGAGAAAGCACTTACAGATCAAAATG
GTGTCCTGCAAAAGCTCATGGAGGCAGAGAAGGAGAAAAACAATGCTTTAATGAACGCACAC
CTCCGGGAGCAGCAAAATG**T**AGCATCAACAAGCTCCCCATCACTGTCACCACCAATGGTTCC
AGATTCCATGCCAACTCTAAATATTGGGTCATGTCAACCTAGAGGGCCAGGAGAATCTGAGC
CTGAACCGTCTCCTGCTCCTGTACAAGCAAACAGCGGCAACCTGCCACCATGGATGCTCCGC
ACTGTCAGTAACAGATGA

**seq id no 17: Saccharum officinarum ZMM28-like reconstituted protein, version 2 (change with seq id no 15 in bold)**

MGSGPVQLRRIENKINRQVTFSKRRNGLLKKAHEISVLCDAEVALIVFSTKGKLYEYSSHSS
MEGILERYQRYSFEERAVLDPSIEDQADWGDEYVRLKSKLDALQKSQRHLLGEQLDSLTIKE
LQQLEQQLDSSLKHIRSRKNQLMFDSISELQKKEKALTDQNGVLQKLMEAEKEKNNALMNAH
LREQQN**V**ASTSSPSLSPPMVPDSMPTLNIGSCQPRGPGESEPEPSPAPVQANSGNLPPWMLR
TVSNR

**seq id no 18: amino acid consensus monocot RT C-terminal motif**
LPPWMLRT

**seq id no 19: amino acid consensus monocot SH C-terminal motif**
LPPWMLSH

**seq id no 20: nucleotide sequence of primer prm05821**
GGGGACAAGTTTGTACAAAAAAGCAGGCTTAAACAATGGGGAGAGGGCCG

**seq id no 21: nucleotide sequence of primer prm05822**
GGGGACCACTTTGTACAAGAAAGCTGGGTTGAGTGGAGTGACGTTTGAGA

**FIGURE 5 (continued)**

**seq id no 22: OSH1 promoter nucleotide sequence**

GGGGAGTTAGGAACCTTGACATACAACCAATGATACCATTTTTTTTCAAGCAGCTAGAGGTA
CAGAGGTTCATATTTTTTAGATGGCTCATTAGTGATATTTTAGTCAAAAATTTCAGAAGTAC
GGCCACGGGTGATGGCCTGAACTAATATTTTATTCGAGGTGCCGCTACATCATCGTCTAAAG
TACACGCAAGATTCAACGGAAAAAAGAAACCGATCGATCGAGATCAGTTAGTTAATGAAATA
ACTAGATCAACTCATGTCGTCAAAAACAAAAGATGCTCATCTATGGACAACACACGCTGATG
ATTCGATCATCAAACAAAGGTGGTAGTAGTAGTAAAGCGTATCGTGTTTCTCATCAGAAAGA
AGAATTAAAGAAAAAACTAATCCCGTCTCGCGAGCCAGAGAAAATTCCCTACAAAAGCCACT
CCTTTGATTTGACATGCAAAAGCAAGGCTCCACTCCTCTACTACCCTACAACTACACAACAC
TGTCTCTCTATCTCCAAAGGCAGTAGCTGTATTGGCTTCCAGCTTTTCCTCTCTACCTCTAA
TGATAGCTTGGAGCAAGTTCAATAGTATAGCTAACTACTAGCTCTAATTCATCTATAATCAA
TCTAATAGCTTATTCATACAATAGTTATATACTACATTATTAATATCTGGTCCCATCTATCA
TACACACTGAGTCTGTGCTATAGCTGACTACAAATCTGTAGCCCGCTGCTCTTCTCTCTTCA
TTTATCTTCTTAAAATATATTTGCAGCTGGCTTATGGCTTATAGCTTGATATTGAGAGGGAG
AGGAGTGAGAGCTAGCTCAGCTCAGCTCAAGGTAAACAACAAGGCACACTCTTCCTACCTCT
TCTCCGGTTCTTCCTTTTTCCTCTTTCTCTTCGTCCAAGAACTTCACCTCAATAGCTCGAGC
TACGGCCTAACTTTTGCGTTGCGCAGGAGAGCTCGATCGCTGCACCAATACTTCACTGGAGA
TCGCCTAGCTGCAGCTAGCTAGCTTATCCTGCGTGTCTTGAGCTTCTCTC

**FIGURE 5 (continued)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6229068 B1
- WO 0233091 A
- WO 03057877 A
- US 5811238 A
- US 6395547 A
- US 5565350 A, Kmiec
- US 9303868 W, Zarling
- EP 1198985 A1
- EP 04106065 A
- US 60632273 B


### Non-patent literature cited in the description


- **JACK.** *Plant Molec Biol,* 2001, vol. 46, 515-520
- **THIESSEN et al.** *J Mol Evol,* 1996, vol. 43, 484-516
- **COEN ; MEYEROWITZ.** *Nature,* 1991, vol. 353, 31-7
- **LITT ; IRISH.** *Genetics,* 2003, vol. 165, 821-833
- **BECKER ; THIESSEN.** *Mol Phylogenet Evol,* 2003, vol. 29 (3), 464-89
- **MASIERO et al.** *J Biol Chem,* 2002, vol. 277 (29), 26429-35
- **FORNARA et al.** *Plant Physiol,* 2004, vol. 135 (4), 2207-19
- **WEST et al.** *Nucl Acid Res,* 1998, vol. 26 (23), 5277-87
- **FIELDS ; SONG.** *Nature,* 1989, vol. 340, 245-6
- **NEEDLEMAN ; WUNSCH.** *J Mol Biol,* 1970, vol. 48, 443-453
- **ALTSCHUL et al.** *J Mol Biol,* 1990, vol. 215, 403-10
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **HAYASHI et al.** *Science,* 1992, 1350-1353
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- current protocols in molecular biology
- **CASTLE et al.** *Science,* 2004, vol. 304 (5674), 1151-4
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **CREIGHTON.** Proteins. W.H. Freeman and Company, 1984
- **BUCHMAN ; BERG.** *Mol. Cell biol.,* 1988, vol. 8, 4395-4405
- **CALLIS et al.** *Genes Dev.,* 1987, vol. 1, 1183-1200
- The Maize Handbook. Springer, 1994
- **MATSUOKA et al.** *Plant Cell,* 1993, vol. 5, 1039-1048
- **SATO et al.** *Proc Natl Acad Sci U S A,* 1996, vol. 93 (15), 8117-22
- **SATO et al.** *PNAS,* 1996, vol. 93, 8117-8122
- **HAKE et al.** *EMBO Journal,* 1989, vol. 8, 15-22
- **LINCOLN et al.** *Plant Cell,* 1994, vol. 6, 1859-1876
- **POSTMA-HAARSMA et al.** *Plant Mol Biol,* 1999, vol. 39 (2), 257-71
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **ALDEMITA ; HODGES.** *Planta,* 1996, vol. 199, 612-617

- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BEMATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- **AUSUBEL et al.** Current Protocols in Molecular Biology. Current Protocols, 1994, vol. 1, 2
- **R.D.D. CROY.** Plant Molecular Biology Labfase. BIOS Scientific Publications Ltd (UK, 1993